Europäisches Patentamt
European Patent Office
Office européen des brevets

(19)

(11) Veröffentlichungsnummer: **0 252 876**
**A1**

(12) # EUROPÄISCHE PATENTANMELDUNG

(21) Anmeldenummer: **87810349.8**

(22) Anmeldetag: **22.06.87**

(51) Int. Cl.4: **C 07 D 211/60**
C 07 D 211/78

(30) Priorität: **26.06.86 CH 2587/86**

(43) Veröffentlichungstag der Anmeldung:
**13.01.88 Patentblatt 88/02**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Züst, Armin, Dr.**
**Sternengasse 27/408**
**CH-4051 Basel (CH)**

(54) **Hydrierte 1-Phenoxyalkylpyridin-3-carbonsäureverbindungen.**

(57) Die Erfindung betrifft neue hydrierte 1-Phenoxyalkylpyridin-3-carbonsäureverbindungen der Formel

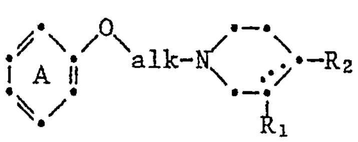

(I),

worin $R_1$ Niederalkoxycarbonyl, Carbamyl, N-Niederalkylcarbamyl, N,N-Diniederalkylcarbamyl oder gegebenenfalls acyliertes Hydroxymethyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, und ihre Tautomeren und/oder Salze. Diese Verbindungen können als pharmazeutische Wirkstoffe verwendet werden und sind in an sich bekannter Weise herstellbar.

EP 0 252 876 A1

**Beschreibung**

Hydrierte 1-Phenoxyalkylpyridin-3-carbonsäureverbindungen

Die Erfindung betrifft neue hydrierte 1-Phenoxyalkylpyridin-3-carbonsäureverbindungen Formel

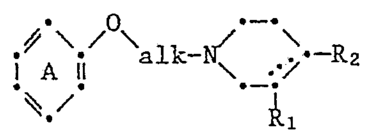

(I),

worin $R_1$ Niederalkoxycarbonyl, Carbamyl, N-Niederalkylcarbamyl, N,N-Diniederalkylcarbamyl oder gegebenenfalls acyliertes Hydroxymethyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, ihre Tautomeren und/oder Salze, die Verwendung dieser Verbindungen, Verfahren zu ihrer Herstellung und pharmazeutische Zusammensetzungen, enthaltend eine Verbindung der Formel I oder ein Tautomeres und/oder ein pharmazeutisch verwendbares Salz davon.

Veräthertes Hydroxy $R_2$ ist beispielsweise Niederalkoxy oder gegebenenfalls substituiertes Phenylniederalkoxy.

Acyl in acyliertem Hydroxymethyl $R_1$ sowie in acyliertem Hydroxy bzw. Amino $R_2$ ist beispielsweise von einer organischen Carbon- oder Sulfonsäure abgeleitetes Acyl.

Von einer organischen Carbonsäure abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl.

Von einer organischen Sulfonsäure abgeleitetes Acyl ist beispielsweise Niederalkansulfonyl.

Der Erfindung betrifft beispielsweise Verbindungen der Formel I, worin $R_1$ Niederalkoxycarbonyl, Carbamyl, N-Niederalkylcarbamyl oder N,N-Diniederalkylcarbamyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, ihre Tautomeren und/oder Salze, die Verwendung dieser Verbindungen, Verfahren zu ihrer Herstellung und pharmazeutische Zussammensetzungen, enthaltend eine solche Verbindung der Formel I oder ein Tautomeres und/oder ein pharmazeutisch verwendbared Salz davon.

Tautomere Formen von Verbindungen der Formel I existieren z.B. dann, wenn $R_2$ Hydroxy oder Amino bedeutet und die gestrichelte Linie zum Ausdruck bringen soll, dass eine Doppelbindung vorliegt. D.h., die Enole bzw. Enamine der Formel I stehen im Gleichgewicht mit den entsprechenden Keto- bzw. Ketimin-Tautomeren der Formel

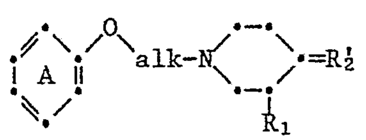

(I'),

worin $R_2'$ Oxo oder Imino bedeutet. Vertreter beider tautomerer Formen können isoliert werden.

Die erfindungsgemässen Verbindungen können ferner in Form von Stereoisomeren vorliegen. Da die Verbindungen der Formel I, falls die gestrichelte Linie für eine Einfachbindung steht, mindestens ein chirales Kohlenstoffatom (C-Atom) besitzen (z.B. das Atom $C_3$ des Piperidinrestes), können sie z.B. als reine Enantiomere oder Enantiomerengemische, wie Racemate, und, sofern noch mindestens ein weiteres chirales Zentrum vorhanden ist (z.B. das Atom $C_4$ eines 4-substituierten Piperidinrestes), auch als Diastereomere, Diastereomerengemische oder Racematgemische vorliegen. So können beispielsweise im Hinblick auf $R_1$ und $R_2$ geometrische Isomere, wie cis-, d.h. 3R,4S- und 3S,4R-, und trans-, d.h. 3R,4R- und 3S,4S-Isomere, gebildet werden, falls $R_2$ von Wasserstoff verschieden ist.

Salze von Verbindungen der Formel I bzw. deren Tautomeren sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensäure, oder mit Sulfonsäuren, wie Niederalkan- oder gegebenenfalls substituierten Benzolsulfonsäuren, z.B. Methan- oder p-Toluolsulfonsäure, gebildet. Bedeutet $R_1$ beispielsweise Hydroxymethyl, können entsprechende Verbindungen Salze mit Basen bilden. Geeignete

Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Triniederalkylamine, wie Hydroxyniederalkylamine, z.B. Mono-, Di- bzw. Trihydroxyniederalkylamine, Hydroxyniederalkyl-niederalkyl-amine oder Polyhydroxyniederalkylamine. Cyclische Amine sind z.B. Morpholin, Thiomorpholin, Piperidin oder Pyrrolidin. Als Mononiederalkylamine kommen beispielsweise Aethyl- oder t-Butylamin, als Diniederalkylamin beispielsweise Diäthyl- oder Diisopropylamin, als Triniederalkylamine beispielsweise Trimethyl- oder Triäthylamin in Betracht. Entsprechende Hydroxyniederalkylamine sind z.B. Mono-, Di- bzw. Triäthanolamin, und Hydroxyniederalkyl-niederalkyl-amine sind z.B. N,N-Dimethylamino- oder N,N-Diäthylamino-äthanol, als Polyhydroxyniederalkylamin kommt z.B. Glucosamin in Frage.

Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Vor- und nachstehend sind unter mit "Nieder" bezeichneten Resten oder Verbindungen, sofern nicht abweichend definiert, insbesondere solche zu verstehen, die bis und mit 7, vor allem bis und mit 4, Kohlenstoffatome enthalten.

Niederalkoxy ist z.B. $C_1$ -$C_4$ -Alkoxy, wie Methoxy, Aethoxy, n-Propyloxy, Isopropyloxy, n-Butyloxy, Isobutyloxy und tert-Butyloxy.

Niederalkyl ist z.B. $C_1$ -$C_4$ -Alkyl, wie Methyl, Aethyl, n-Propyl, Isopropyl, n-Butyl, Isobutyl, sek-Butyl oder tert-Butyl und umfasst ferner $C_5$ -$C_7$ -Alkyl-, wie Pentyl-, Hexyl- und Heptylreste.

Niederalkylen alk ist z.B. $C_1$ -$C_4$ -Alkylen, welches die beiden in Formel I eingezeichneten Heteroatome O und N in erster Linie durch 2 bis und mit 4 C-Atome überbrückt, und kann z.B. Aethylen, 1,3-Propylen oder 1,4-Butylen, aber auch Methylen, 1,2-Propylen, 1,2- oder 1,3-(2-Methyl)-propylen oder 1,2- oder 1,3-Butylen sein.

Niederalkanoyl ist z.B. $C_2$ -$C_5$ -Alkanoyl, wie Acetyl, Propionyl, Butyryl, Isobutyryl oder Pivaloyl.

Niederalkanoyloxy ist z.B. $C_2$ -$C_5$ -Alkanoyloxy, wie Acetyloxy, Propionyloxy, Butyryloxy, Isobutyryloxy oder Pivaloyloxy.

Niederalkoxycarbonyl ist z.B. $C_2$ -$C_5$ -Alkoxycarbonyl, wie Methoxy-, Aethoxy-, n-Propyloxy-, Isopropyloxy-, n-Butyloxy-, Isobutyloxy-oder tert.-Butyloxycarbonyl.

N-Niederalkylcarbamyl ist z.B. N-$C_1$ -$C_4$ -Alkylcarbamyl, wie N-Methyl-, N-Aethyl-, N-(n-Propyl)-, N-Isopropyl-, N-(n-Butyl)-, N-Isobutyl-oder N-tert.-Butylcarbamyl.

N,N-Diniederalkylcarbamyl ist z.B. N,N-Di-$C_1$ -$C_4$ -alkylcarbamyl, wobei jeweils die beiden N-Alkylgruppen gleich oder verschieden sein können, wie N,N-Dimethyl-, N,N-Diäthyl-, N,N-Diisopropyl- oder N-Butyl-N-methylcarbamyl.

Gegebenenfalls substituiertes Phenylniederalkoxy ist z.B. gegebenenfalls im Phenylteil substituiertes Phenyl-$C_1$ -$C_4$ -alkoxy, wie Benzyloxy, p-Chlorbenzyloxy, 1-Phenyläthoxy oder 1-(p-Bromphenyl)-n-butyloxy.

Gegebenenfalls substituiertes Benzoyl ist z.B. Benzoyl, p-Chlorbenzoyl oder p-Nitrobenzoyl.

Niederalkansulfonyl ist z.B. $C_1$ -$C_4$ -Alkansulfonyl, wie Methan- oder Aethansulfonyl.

Halogen ist insbesondere Halogen mit einer Atomnummer bis und mit 35, wie Fluor, Chlor und Brom, und umfasst ferner Iod.

Die Verbindungen der Formel I, ihre Tautomeren und/oder ihre pharmazeutisch verwendbaren Salze weisen beispielsweise wertvolle pharmakologische, insbesondere nootrope, Eigenschaften auf. So bewirken sie z.B. im Two-Compartment Passive Avoidance-Testmodell nach Mondadori und Classen, Acta Neurol. Scand. 69, Suppl. 99, 125 (1984) in Dosismengen ab etwa 0,1 mg/kg i.p. sowie p.o. bei der Maus eine Reduktion der amnesischen Wirkung eines zerebralen Elektroschocks.

Ebenso besitzen die erfindungsgemässen Verbindungen eine beträchtliche gedächtnisverbessernde Wirkung, die im Step-down Passive Avoidance-Test nach Mondadori und Waser, Psychopharmacol. 63, 297 (1979) ab einer Dosis von etwa 0,1 mg/kg i.p. sowie p.o. an der Maus festzustellen ist.

Entsprechend können die Verbindungen der Formel I bzw. deren Tautomere und/oder ihre pharmazeutisch verwendbaren Salze als Pharmazeutika, z.B. Nootropika, beispielsweise zur therapeutischen und/oder prophylaktischen Behandlung von cerebralen Insuffizienzerscheinungen, insbesondere Gedächtnisstörungen, verwendet werden. Ein weiterer Gegenstand der Erfindung ist somit die Verwendung von Verbindungen der Formel I, ihrer Tautomeren und/oder ihrer pharmazeutisch verwendbaren Salze zur Herstellung von Arzneimitteln, insbesondere von Nootropika, zur Behandlung von cerebralen Insuffizienzerscheinungen, insbesondere Gedächtnisstörungen. Dabei kann auch die gewerbsmässige Herrichtung der Wirksubstanzen eingeschlossen sein.

Die Erfindung betrifft in erster Linie Verbindungen der Formel I, worin $R_1$ Niederalkoxycarbonyl, Carbamyl, N-Niederalkylcarbamyl, N,N-Diniederalkylcarbamyl, Hydroxymethyl, Niederalkanoyloxymethyl, Niederalkansulfonyloxymethyl, Benzoyloxymethyl oder Pyridoyloxymethyl bedeutet, $R_2$ Wasserstoff, Hydroxy, Niederalkoxy, Benzyloxy, Nideralkanoyloxy, Niederalkansulfonyloxy, Benzoyloxy, Pyridoyloxy, Amino, Niederalkanoylamino, Niederalkansulfonylamino, Benzoylamino oder Pyridoylamino darstellt, alk Niederalkylen, das die beiden in Formel I eingezeichneten Heteroatome O und N in erster Linie durch 2 bis und mit 4 C-Atome überbrückt, bedeutet, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, beispielsweise

Verbindungen der Formel I, worin $R_1$ Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Benzoylamino oder Pyridoylamino darstellt, alk Niederalkylen, das die beiden in Formel I eingezeichneten Heteroatome O und N in erster Linie durch 2 bis und mit 3 C-Atome überbrückt, bedeutet, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, und ihre Tautomeren und/oder Salze.

Die Erfindung betrifft vor allem Verbindungen der Formel I, worin $R_1$ $C_1$-$C_4$-Alkoxycarbonyl, wie Methoxycarbonyl, Aethoxycarbonyl, n-Butyloxycarbonyl oder tert.-Butyloxycarbonyl, Carmamyl, N-$C_1$-$C_4$-Alkylcarbamyl, wie N-Methylcarbamyl, N,N-Di-$C_1$-$C_4$-Alkylcarbamyl, wie N,N-Diäthylcarbamyl, Hydroxymethyl oder $C_2$-$C_5$-Alkanoyloxymethyl, wie Acetoxymethyl, bedeutet, $R_2$ Wasserstoff, Hydroxy oder Amino bedeutet, alk für $C_1$-$C_4$-Alkylen, das die beiden in Formel I eingezeichneten Heteroatome O und N in erster Linie durch 2 bis mit 4 C-Atome überbrückt, wie Aethylen, 1,3-Propylen oder 1,4-Butylen, steht, der Ring A unsubstituiert oder ein-, zwei- oder mehrfach durch $C_1$-$C_4$-Alkoxy, wie Methoxy, Cyano, Halogen mit einer Atomnummer bis und mit 35, wie Fluor und/oder Chlor, $C_1$-$C_4$-Alkyl, wie Methyl, und/oder Trifluormethyl substituiert ist und die gestrichelte Linie zum Ausdruck bringen soll, dass eine Einfach- oder eine Doppelbindung vorliegt, beispielsweise Verbindungen der Formel I, worin $R_1$ $C_1$-$C_4$-Alkoxycarbonyl, wie Methoxycarbonyl, oder Carbamyl bedeutet, $R_2$ Wasserstoff oder Hydroxy bedeutet, alk für $C_1$-$C_4$-Alkylen, das die beiden in Formel I eingezeichneten Heteroatome O und N in erster Linie durch 2 bis und mit 3-C-Atome überdrückt, wie Aethylen oder 1,3-Propylen, steht, der Ring A unsubstituiert oder ein-, zwei- oder mehrfach durch $C_1$-$C_4$-Alkoxy, wie Methoxy, Cyano, Halogen mit einer Atomnummer bis und mit 35, wie Fluor und/oder Chlor, $C_1$-$C_4$-Alkyl, wie Methyl, und/oder Trifluormethyl substituiert ist und die gestrichelte Linie zum Ausdruck bringen soll, dass eine Doppelbindung vorliegt, und ihre Tautomeren und/oder Salze.

Die Erfindung betrifft insbesondere Verbindungen der Formel I, worin $R_1$ $C_1$-$C_4$-Alkoxycarbonyl, wie Methoxycarbonyl oder Aethoxycarbonyl, oder Carbamyl bedeutet, $R_2$ Wasserstoff oder Hydroxy darstellt, alk für $C_1$-$C_4$-Alkylen, das die beiden in Formel I eingezeichneten Heteroatome O und N in erster Linie durch 2 bis und mit 3 C-Atome überbrückt, wie Aethylen oder 1,3-Propylen, steht, der Ring A einfach durch $C_1$-$C_4$-Alkoxy, wie Methoxy, $C_1$-$C_4$-Alkyl, wie Methyl, oder Trifluormethyl oder zweifach durch Halogen mit einer Atomnummer bis und mit 35, wie Chlor, oder $C_1$-$C_4$-Alkyl, wie Methyl, oder $C_1$-$C_4$-Alkoxy, wie Methoxy, sowie $C_1$-$C_4$-Alkyl, wie Methyl, substituiert ist und die gestrichelte Linie zum Ausdruck bringen soll, dass eine Einfach- oder eine Doppelbindung vorliegt, und ihre Tautomeren und/oder Salze.

Die Erfindung betrifft in allererster Linie Verbindungen der Formel I, worin $R_1$ $C_1$-$C_4$-Alkoxycarbonyl, wie Methoxycarbonyl, bedeutet, $R_2$ Wasserstoff darstellt, alk für Aethylen steht, der Ring A einfach durch $C_1$-$C_4$-Alkoxy, wie Methoxy, oder Trifluormethyl oder zweifach durch Halogen mit einer Atomnummer bis und mit 35, wie Chlor, substituiert ist und die gestrichelte Linie zum Ausdruck bringen soll, dass eine Doppelbindung vorliegt, und ihre Tautomeren und/oder Salze.

Die Erfindung betrifft namentlich die in den Beispielen genannten neuen Verbindungen und Verfahren zu ihrer Herstellung.

Ebenfalls Gegenstand der vorliegenden Erfindung sind Verfahren zur Herstellung von Verbindungen der Formel I, ihrer Tautomeren und ihrer Salze, z.B. dadurch gekennzeichnet, dass man

a) ein Verbindung der Formel

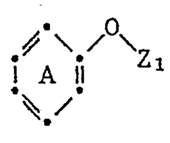

(IIa)

oder ein Salz davon mit einer Verbindung der Formel

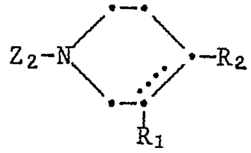

(IIb),

einem Tautomeren und/oder Salz davon, wobei entweder $Z_1$ eine Gruppe alk-$X_1$ und $Z_2$ Wasserstoff ist, oder $Z_1$ Wasserstoff und $Z_2$ eine Gruppe alk-$X_1$ darstellt, jeweils mit $X_1$ als Hydroxy oder reaktionsfähigem veresterten Hydroxy, umsetzt oder

b) in einer Verbindung der Formel

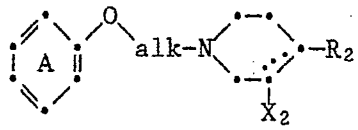

(III) ,

einem Tautomeren und/oder Salz davon, worin $X_2$ einen in $R_1$ überführbaren Rest bedeutet, $X_2$ in $R_1$ überführt oder

c) zur Herstellung einer Verbindung der Formel I, eines Tautomeren und/oder Salzes davon, worin $R_2$ Hydroxy oder Amino bedeutet, und worin $R_1$ insbesondere Niederalkoxycarbonyl bedeutet, eine Verbindung der Formel

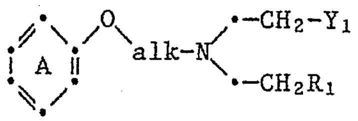

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', eines Tautomeren und/oder Salzes davon, worin $R_2'$ Oxo oder Imino bedeutet und die gestrichelte Linie zum Ausdruck bringen soll, dass eine Einfachbindung vorliegt, und worin $R_1$ insbesondere Niederalkoxycarbonyl bedeutet, eine Verbindung der Formel

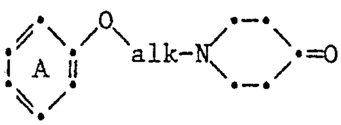

(Va)

oder ein Tautomeres oder jeweils ein Salz davon mit einer Verbindung der Formel

$X_3-R_1$ (Vb)

oder einem Salz davon, worin $X_3$ Halogen oder Niederalkoxy bedeutet, umsetzt oder

e) zur Herstellung einer Verbindung der Formel I, eines Tautomeren und/oder Salzes davon, worin $R_2$ von Wasserstoff verschieden ist, in einer Verbindung der Formel

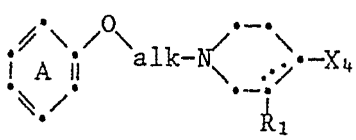

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I, eines Tautomeren und/oder Salzes davon, worin $R_2$ Wasserstoff bedeutet, eine Verbindung der Formel

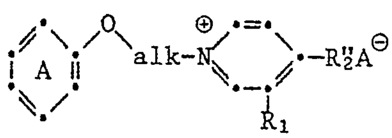

(VII),

worin $A^{\ominus}$ für das Anion einer Säure steht, und $R_2''$ Wasserstoff, veräthertes, verestertes oder geschütztes Hydroxy oder acyliertes oder geschütztes Amino bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung in eine andere Verbindung der Formel I überführt, jeweils ein verfahrensgemäss erhältliches Isomerengemisch in die Komponenten auftrennt, jeweils ein verfahrensgemäss erhältliches Enantiomeren bzw. Diastereomerengemisch in die Enantiomeren bzw. Diastereomeren aufspaltet, jeweils eine verfahrensgemäss erhältliche freie Verbindung der Formel I in ein Salz überführt und/oder jeweils ein verfahrensgemäss erhältliches Salz in die freie Verbindung der Formel I oder in ein anderes Salz umwandelt.

Die vor- und nachstehend in den Varianten beschriebenen Umsetzungen werden in an sich bekannter Weise

durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen z.B. in einem Temperaturbereich von etwa -10° bis zur Siedetemperatur des Reaktionsmediums, vorzugsweise von etwa 20° bis etwa 150°C, und, falls erforderlich, in einem geschlossenen Gefäss, unter Druck, in einer Inertgasatmosphäre und/oder unter wasserfreien Bedingungen arbeitet.

Das vor- und nachstehend aufgeführte Ausgangsmaterial der Formeln IIa und IIb, III, IV, Va und Vb, VI und VII, das für die Herstellung der Verbindungen der Formel I, deren Tautomeren und Salzen entwickelt wurde, ist zum Teil bekannt oder kann ebenfalls nach an sich bekannten Methoden, z.B. analog den vorstehend beschriebenen Verfahrensvarianten, hergestellt werden.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen, Salze mit Basen, z.B. der vorstehend genannten Art, bilden können. Weiter können Ausgangsverbindungen in Form von Tautomeren vorliegen, insbesondere bei Verbindungen der Formel IIb, wenn $R_2$ Hydroxy bedeutet und die gestrichelte Linie zum Ausdruck bringen soll, dass eine Doppelbindung vorliegt.

Variante a):

Reaktionsfähiges verestertes Hydroxy bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Die N-Alkylierung wird insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, - diniederalkylamide, -aminoniederalkylamide oder - niederalkylsilylamide, Naphthalinamine, Niederalkylamine, basische Heterocyclen, Ammoniumhydroxide sowie carbocyclische Amine in Frage. Beispielhaft seien Natriumhydroxid, - hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethyl-ammoniumhydroxid, 1,5-Diaza-bicyclo[4.3.0]non-5-en (DBN) sowie 1,5-Diazo-bicyclo[5.4.0]undec-5-en (DBU) genannt.

Die Ausgangsmaterialien der Formeln IIa und IIb sind teilweise bekannt oder lassen sich in Analogie zu den bekannten Ausgangsmaterialien herstellen.

Variante b):

Ein in $R_1$ überführbarer Rest $X_2$ ist beispielsweise von $R_1$ verschiedenes funktionell abgewandeltes Carboxy, wie Cyano, anhydridisiertes Carboxy oder von verestertem oder amidiertem Carboxy $R_1$ verschiedenes verestertes oder amidiertes Carboxy.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Kohlensäurehalogenid-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

$X_2$ kann beispielsweise durch Solvolyse in $R_1$ überführt werden. Solvolysemittel sind beispielsweise dem gewünschten veresterten Carboxy $R_1$ entsprechende Niederalkanole, Ammoniak oder der gewünschten amidierten Carboxygruppe $R_1$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren, z.B. Schwefelsäure oder Halogenwasserstoffsäuren, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder wie Carbonsäuren, z.B. Niederalkancarbonsäuren, beispielsweise Essigsäure, in Frage, während als Basen beispielsweise die unter Variante a) genannten verwendet werden können, insbesondere Natrium- oder Kaliumhydroxid.

Cyano, anhydridisiertes Carboxy und von verestertem oder amidiertem Carboxy $R_1$ verschiedenes verestertes oder amidiertes Carboxy werden beispielsweise mit einem geeigneten Niederalkanol zu verestertem Carboxy $R_1$ alkoholysiert und Cyano und anhydridisiertes Carboxy beispielsweise mit Ammoniak oder einem dem amidierten Carboxy $R_1$ entsprechenden Amin ammono- bzw. aminolysiert.

Das Ausgangsmaterial der Formel III kann in Analogie zu der unter Variante a) beschriebenen Weise durch Umsetzung einer Verbindung der Formel

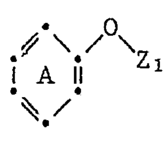

(IIa)

mit einer Verbindung der Formel

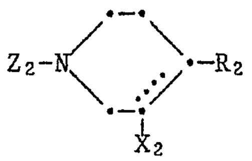

(IIIa),

einem Tautomeren und/oder Salz davon in Gegenwart einer der genannten Basen erfolgen.

Variante C:

Abspaltbare Reste $Y_2$ in Gruppen der Formel $-C(Y_2)=R_2'$ oder $-CH(Y_2)-R_2$ sind z.B. verätherte oder reaktionsfähige veresterte Hydroxygruppen, wie Niederalkoxy, Halogen oder Sulfonyloxy.

Die Cyclisierung kann beispielsweise in Analogie zur Dieckmann-Reaktion, insbesondere in Gegenwart einer der unter der Variante a) genannten Basen und unter anschliessender hydrolytischer Aufarbeitung durchgeführt werden.

In einer bevorzugten Ausführungsform kann man beispielsweise eine Verbindung der Form

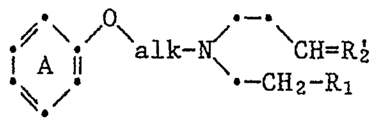

(IVa),

worin $R_2'$ Oxo oder Imino bedeutet, der Behandlung mit einer der genannten Basen, insbesondere mit einem Alkalimetallniederalkanolat, z.B. mit Natriummethanolat oder Natriumäthanolat, unterwerfen. Dabei cyclisiert die Verbindung IVa zu einer Verbindung der Formel I, worin die gestrichelte Linie anzeigt, dass keine Doppelbindung vorliegt und $R_2$ Hydroxy oder Amino bedeutet. Ausgangsstoffe IVa werden z.B. erhalten, indem man einen reaktionsfähigen Phenoxyäthylester der Formel

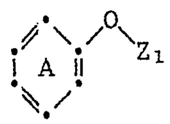

(IIa),

worin $Z_1$ eine Gruppe der Formel alk-$X_1$ bedeutet, in welcher $X_1$ reaktionsfähiges verestertes Hydroxy ist, mit einer Verbindung der Formel $H_2N-CH_2-CH_2-R_1$ (IVb) und das erhaltene Zwischenprodukt der Formel

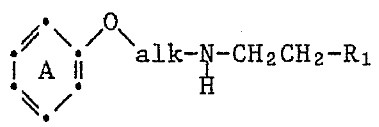

(IVc)

mit Acrolein oder einem gegebenenfalls funktionell abgewandelten Aldehyd der Formel $Y_1-CH_2-CH_2-CH=R_2'$ (IVd; $Y_1$ = reaktionsfähiges verestertes Hydroxy; $R_2'$ = Oxo oder Imino) umsetzt.

In einer anderen bevorzugten Ausführungsform der Variante c) cyclisiert man eine Verbindung der Formel IV, worin $Y_1$ und $R_1$ Niederalkoxycarbonyl bedeuten, d.h. worin $Y_1$ eine Gruppe der Formel $-C(Y_2)=R_2'$ bedeutet, in welcher $R_2'$ Oxo ist und der abspaltbare Rest $Y_2$ als veräthertes Hydroxy eine Niederalkoxygruppe aufweist, zu der entsprechenden Verbindung der Formel I', worin $R_2'$ Oxo ist.

Zur Herstellung der letztgenannten Ausgangsverbindungen der Formel IV kann man beispielsweise von Verbindungen der Formel

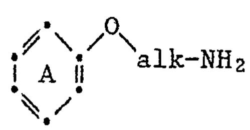

(IVe)

oder deren Salzen ausgehen, welche z.B. durch Reduktion der entsprechenden Nitrile erhältlich sind, und diese mit mindestens 2 Mol einer Verbindung der Formel

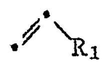

(IVf)

zur Reaktion bringen.

Variante d):

Die verfahrensgemässe C-Acylierung kann insbesondere in Gegenwart einer der unter der Variante a) genannten Basen erfolgen.

Die Umsetzung einer Verbindung der Formel

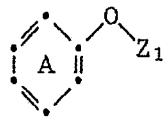

(IIa)

mit einer Verbindung der Formel

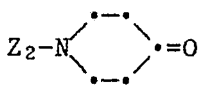

(Vc)

oder einem Salz davon führt in Analogie zu der N-Alkylierung gemäss Variante a) in Gegenwart einer der erwähnten Basen zu dem Ausgangsmaterial der Formel Va.

Variante e):

In $R_2$ überführbare Reste $X_4$ sind beispielsweise durch Solvolyse, d.h. Umsetzung mit einer Verbindung der Formel $R_2H$ oder einem Salz davon, in eine Gruppe $R_2$ überführbare Reste, beispielsweise reaktionsfähige veresterte Hydroxygruppen, wie Halogenatome, z.B. Chlor, Brom oder Iod. In Hydroxy überführbare Reste $X_4$ sind ferner Diazoniumgruppen, z.B. der Formel $-N_2{}^{\oplus}A^{\ominus}$, worin $A^{\ominus}$ das Anion einer starken Säure, wie einer Mineralsäure, z.B. das Chlorid- oder Sulfation, bedeutet.

Die Solvolyse erfolgt in üblicher Weise, beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triäthylamin, oder einer heteroaromatischen Stickstoffbase, wie Pyridin, bzw. eines quaternären Ammoniumhydroxides, wie Benzyl-trimethyl-ammoniumhydroxid, oder indem man die Verbindung VIa in Form eines Metallsalzes, z.B. der Formel $R_2{}^{\ominus}M^{\oplus}$ (VIb), worin $M^{\oplus}$ ein Alkalimetallkation, wie das Natriumion, bedeutet, einsetzt. Vorteilhaft arbeitet man in Gegenwart eines Lösungs- oder Verdünnungsmittels, z.B. in einem Ueberschuss der Reaktionskomponente VIa und/oder einem mit dieser mischbaren inerten Lösungsmittel, erforderlichenfalls unter Kühlen oder Erwärmen, z.B. im Temperaturbereich von etwa 0° bis 120°C, und/oder unter Inertgas, wie Stickstoff.

Die Solvolyse von Resten $X_4$ zu Gruppen $R_2$ gegebenenfalls mit der solvolytischen Ueberführung von solvolysierbaren Gruppen $R_1$ in andere erfindungsgemässe Gruppen $R_1$ kombiniert werden, beispielsweise können bei der Ammonolyse von Resten $X_4$ zu Amino $R_2$ gegebenenfalls auch Niederalkoxycarbonylgruppen $R_1$ oder andere zu Carbamyl $R_1$ solvolysierbare Gruppen $R_1$ gleichzeitig zu Carbamylgruppen $R_1$ ammonolysiert werden.

Zur Herstellung von Ausgangsverbindungen der Formel VI und deren Salzen geht man beispielsweise von Verbindungen der Formel IIa

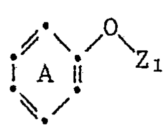

(IIa)

aus und setzt diese mit einer entsprechenden Verbindung der Formel

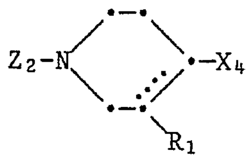

(VIa)

oder einem Salz davon in Gegenwart einer der vorstehend genannten Basen um, wobei z.B. in analoger Weise wie unter der Verfahrensvariante a) beschrieben verfahren wird.

In einer bevorzugten Ausführungsform erhält man Verbindungen VI, worin $X_4$ für Halogen und die gestrichelte Linie für eine Einfachbindung steht, und deren Salze durch Umsetzung einer Verbindung der Formel I, worin $R_2$ Hydroxy bedeutet und die gestrichelte Linie für eine Einfachbindung steht, oder eines Salzes davon mit einem Halogenierungsmittel, wie Phosphortri- oder -pentachlorid oder Thionylchlorid, wobei die entsprechenden Verbindungen I und ihre Salze beispielsweise in analoger Weise wie unter der Verfahrensvariante a) beschrieben erhalten werden können.

Variante f):

Das Anion $A^{\ominus}$ ist beispielsweise das Anion einer starken Protonsäure, z.B. ein Halogenidion, wie Chlorid-, Bromid- oder Iodidion, oder ein Sulfonation, wie ein gegebenenfalls substituiertes Niederalkan- oder Benzolsulfonation, z.B. das Methansulfonat-, Aethansulfonat- oder p-Bromphenylsulfonat- oder p-Toluolsulfonation. $R_2''$ ist insbesondere Wasserstoff, veräthertes Hydroxy $R_2$ oder geschütztes Hydroxy. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Dipheynl- oder Triphenylniederalkylamino, wie Benzylamino, Diphenylamino oder Tritylamino, sein.

Die Reduktion der überschüssigen Doppelbindungen erfolgt durch Behandeln mit einem geeigneten Reduktionsmittel, beispielsweise durch Hydrierung in Gegenwart eines Hydrierungskatalysators, durch Reduktion mit einem Hydrid-übertragenden Reagenz oder durch Reduktion mit einem metallischen Reduktionssystem aus Metall und Protonen-abspaltendem Mittel.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris(triphenylphosphan)-rhodium-I-halogenid, z.B. -chlorid, oder Raney-Nickel, die gegebenenfalls auf einem Trägermaterial, wie Aktivkohle, Alkalimetallcarbonat bzw. -sulfat oder einem Kieselgel aufgezogen sind. Als Hydrid-übertragende Reagenzien kommen beispielsweise geeignete Leichtmetallhydride, insbesondere Alkalimetallaluminiumhydride bzw. -borhydride, wie Lithiumaluminiumhydrid, Lithiumtriäthylborhydrid, Natriumborhydrid, Natriumcyanoborhydrid, oder Zinnhydride, wie Triäthyl- oder Tributylzinnhydrid, oder Diboran in Frage. Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Uebergangsmetall, z.B. Zink, Zinn, Eisen oder Titan, während als Protonen-abspaltende Mittel z.B. Protonsäuren der vorstehend genannten Art, wie Salz- oder Essigsäure, Niederalkanole, wie Aethanol, und/oder Amine bzw. Ammoniak in Frage kommen. Solche Systeme sind beispielsweise Natrium/Ammoniak, Zink/Salz- oder Essigsäure oder Zink/Aethanol.

Die Herstellung von Ausgangsverbindungen der Formel VII erfolgt beispielsweise durch Umsetzung von Verbindungen der Formel

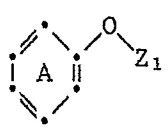

(IIa),

worin $Z_1$ eine Gruppe der Formel alk-A bedeutet, in welcher A dem Anion $A^{\ominus}$ entsprechendes reaktionsfähiges verestertes Hydroxy ist, mit Verbindungen der Formel

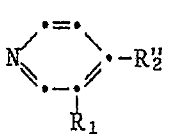

(VIIa)

oder einem Salz davon, wobei z.B. in analoger Weise wie unter der Verfahrensvariante a) beschrieben verfahren wird.

In den Ausgangsstoffen der Formeln IIb, III und IIIa kann eine Hydroxygruppe $R_2$ in verätherter bzw. eine

Hydroxy- oder Aminogruppe $R_2$ auch in intermediär geschützter Form vorliegen. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylmethylamino oder Tritylamino, sein.

Die Freisetzung intermediär geschützter Reste $R_2$, d.h. Abspaltung der intermediären Schutzgruppen, erfolgt in üblicher Weise, beispielsweise durch Solvolyse, wie milde Hydrolyse, z.B. Behandlung mit Wasser unter neutralen oder schwach sauren Bedingungen, z.B. durch Einwirkung von verdünnt-wässrigen Mineral- oder Carbonsäuren, z.B. von verdünnter Salz- oder Essigsäure. In analoger Weise erfolgt die Freisetzung intermediär geschützter Hydroxy- oder Aminogruppen $R_2''$ in Ausgangsstoffen der Formeln VII bzw. VIIa.

Verfahrensgemäss oder anderweitig erhältliche Verbindungen der Formel I können in üblicher Weise in andere Verbindungen der Formel I übergeführt werden.

So kann man z.B. veresterte Carboxygruppen $R_1$ in üblicher Weise durch Umesterung, d.h. Behandlung mit einem Alkohol in Gegenwart eines sauren oder basischen Solvolysemittels, wie einer Mineralsäure, z.B. von Schwefelsäure, bzw. eines entsprechenden Alkalimetallalkoholates oder eines Alkalimetallhydroxides, in andere veresterte Carboxygruppen $R_1$ oder durch Umsetzung mit Ammoniak oder einem entsprechenden mindestens ein Wasserstoffatom aufweisenden Amin in amidiertes Carboxy $R_1$ überführen.

Ferner kann man gegebenenfalls vorhandene Hydroxygruppen verestern, z.B. durch Behandeln mit einem Niederalkancarbonsäureanhydrid bzw. -halogenid in Niederalkanoyloxy überführen oder durch Umsetzung mit einem reaktionsfähigen Ester, insbesondere Brom- oder Chlorwasserstoffsäureester, eines Niederalkanols in entsprechendes veräthertes Hydroxy überführen. Umgekehrt kann man aus verestertem oder veräthertem Hydroxy, wie Niederalkanoyloxy oder Niederalkoxy, die Hydroxygruppe(n) solvolytisch freisetzen, vorzugsweise unter sauren Bedingungen. In analoger Weise kann man auch veräthertes oder acyliertes Hydroxy $R_2$ zu Hydroxy hydrolysieren.

Entsprechend kann man weiterhin Hydroxymethyl $R_1$ verestern, z.B. durch Behandeln mit einem Niederalkancarbonsäureanhydrid bzw. -halogenid in Niederalkanoyloxymethyl $R_1$ überführen. Umgekehrt kann man aus acyliertem Hydroxymethyl, z.B. Niederalkanoyloxymethyl, $R_1$ die Hydroxygruppe solvolytisch freisetzen, vorzugsweise unter sauren Bedingungen.

Weiterhin lässt sich Hydroxymethyl $R_1$ in üblicher Weise in Niederalkoxycarbonyl, Carbamyl, N-Niederalkylcarbamyl oder N,N-Diniederalkylcarbamyl $R_1$ überführen wobei z.B. in der Weise verfahren wird, dass zunächst Hydroxymethyl $R_1$ in üblicher Weise, z.B. in Gegenwart eines Oxidationsmittels, wie Kaliumpermanganat oder Kaliumdichromat, zu Carboxy oxidiert wird und anschliessend die Carboxygruppe in üblicher Weise, z.B. durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triäthylamin, oder durch Ueberführung in ein Alkalimetallsalz und anschliessende Umsetzung mit einem reaktionsfähigen Ester des entsprechenden Alkolhols, wie einem entsprechenden Halogenid, oder unter Verwendung eines Dehydratisierungsmittels, wie N,N'-Dicyclohexylcarbodiimid, mit einem entsprechenden Alkohol in Niederalkoxycarbonyl $R_1$ oder durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ überführt wird. Ebenso kann man acyliertes Hydroxymethyl $R_1$ in verestertes oder amidiertes Carboxy $R_1$ überführen, indem man zunächst die acylierte Hydroxymethylgruppe solvolytisch freisetzt, z.B. wie vorstehend beschrieben, und dann die erhaltene freie Hydroxymethylgruppe, wie vorstehend erläutert, in eine Carboxylgruppe und letztere weiter in eine veresterte oder amidierte Carboxylgruppe überführt. Umgekehrt lassen sich veresterte oder amidierte Carboxygruppen $R_1$ in gegebenenfalls acyliertes Hydroxymethyl $R_1$ überführen, indem man zunächst die veresterte oder amidierte Carboxygruppe $R_1$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy hydrolysiert und anschliessend die erhaltene Carboxygruppe in üblicher Weise, z.B. in Gegenwart eines Reduktionsmittels, beispielsweise der vorstehend erwähnten Art, zu Hydroxymethyl $R_1$ reduziert, wobei gewünschtenfalls letzteres, z.B. wie vorstehend beschrieben, anschliessend noch in acyliertes Hydroxymethyl $R_1$ überführt werden kann.

Bedeutet die gestrichelte Linie, dass in den erfindungsgemässen Verbindungen eine Doppelbindung vorliegt, kann diese z.B. in an sich bekannter Weise mit Hilfe eines Reduktionsmittels, z.B. der unter Variante f) aufgeführten Art, zu einer Einfachbindung hydriert werden.

Ferner kann eine erfindungsgemässe Verbindung, in der die gestrichelte Linie für eine Doppelbindung steht und $R_2$ Wasserstoff bedeutet, z.B. in an sich bekannter Weise durch Addition einer Verbindung $R_2$-H, in der $R_2$ eine gegebenenfalls verätherte oder acylierte Hydroxygruppe oder eine gegebenenfalls acylierte Aminogruppe darstellt, in eine entsprechende erfindungsgemässe Piperidinverbindung überführt werden. Die Addition wird dabei insbesondere in Gegenwart einer geeigneten Base, z.B. der unter Variante a) aufgeführten Art, durchgeführt.

Erfindungsgemässe Verbindungen, in denen die gestrichelte Linie für eine Einfachbindung steht, können umgekehrt z.B. durch Eliminierung einer Verbindung $R_2$-H, in der $R_2$ eine gegebenenfalls verätherte oder

acylierte Hydroxygruppe oder eine gegebenenfalls acylierte Aminogruppe darstellt, in an sich bekannter Weise in entsprechende erfindungsgemässe Tetrahydro-Pyridin-Verbindungen, in denen $R_2$ Wasserstoff darstellt, überführt werden. Für eine Eliminierung schlechter geeignete Abgangsgruppen $R_2$, z.B. Hydroxy, können dabei zuvor, beispielsweise in situ, in besser geeignete Abgangsgruppen $R_2$, z.B. Niederalkansulfonyloxy, wie Methansulfonyloxy, oder Halogen, wie Chlor, Brom oder Iod, umgewandelt werden. Die Eliminierung erfolgt dabei insbesondere in Gegenwart einer geeigneten Base, z.B. der unter Variante a) aufgeführten Art.

Salze von Verbindungen der Formel I bzw. von deren Tautomeren können in an sich bekannter Weise hergestellt werden. So erhält man beispielsweise Säureadditionssalze von Verbindungen der Formel I durch Behandeln mit einer Säure oder einem geeigneten Ionenaustauscherreagens. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenen, insbesondere basischen Eigenschaften, in freier Form oder in Form von Salzen erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbindenden Verbindungen können auch in Form ihrer Hydrate erhalten werden oder andere, beispielsweise zur Kristallisation von in fester Form vorliegenden Verbindungen verwendete, Lösungsmittel einschliessen.

Die neuen Verbindungen können, je nach Wahl der Ausgangsstoffe und Arbeitsweisen, in Form eines der möglichen Isomeren oder als Gemisch derselben vorliegen. Dabei sind, in Abhängigkeit von der Molekülsymmetrie, z.B. je nach Anzahl, absoluter und relativer Konfiguration der Chiralitätszentren, wie asymmetrischen C-Atome, als reine Isomere z.B. reine Enantiomere und/oder reine Diastereomere, wie reine cis/trans-Isomere oder meso-Verbindungen, erhältlich. Entsprechend können also Isomerengemische z.B. Enantiomerengemische, wie Racemate, Diastereomerengemische oder Racematgemische vorliegen.

Erhaltene Diastereomerengemische und Racematgemische können aufgrund der physikalisch-chemischen Unterschiede der Bestandteile in bekannter Weise in die reinen Diastereomeren oder Racemate aufgetrennt werden, beispielsweise durch fraktionierte Kristallisation.

Erhaltene Enantiomerengemische, wie Racemate, lassen sich nach bekannten Methoden in die Enantiomeren zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Enantiomeren verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen $R_1$, $R_2$ und alk sowie die Substituenten des Ringes A die für die jeweils bevorzugten Verbindungsgruppen der Formel I angegebenen Bedeutungen haben.

Die Erfindung betrifft ebenfalls die Verwendung von Verbindungen der Formel I, deren Tautomeren und/oder von pharmazeutisch verwendbaren Salzen von solchen Verbindungen mit salzbildenden Eingenschaften, insbesondere als pharmakologische, in erster Linie nootrop wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Nootropika, z.B. zur Behandlung von cerebralen Insuffizienzerscheinungen, insbesondere Gedächtnisstörungen, anwenden.

Die Erfindung betrifft gleichfalls pharmazeutische Präparate, die eine Verbindung der Formel I oder ein Tautomeres und/oder pharmazeutisch verwendbares Salz davon als Wirkstoff enthalten, sowie Verfahren zu ihrer Herstellung.

Bei den erfindungsgemässen pharmazeutischen Präparaten, welche eine Verbindung der Formel I oder ein Tautomeres und/oder pharmazeutisch verwendbares Salz davon enthalten, handelt es sich um solche zur enteralen, wie oralen, ferner rektalen, und parenteralen Verabreichung an Warmblüter(n), wobei der pharmakologische Wirkstoff allein oder zusammen mit üblichen pharmazeutischen Hilfsstoffen enthalten ist.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder

Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliess-, Regulier-und Schmiermitel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen. Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete, lipophile Lösungsmittel oder Vehikel, wie fette Oele, z.B. Sesamöl, oder synthetische Fettsäureester, z.B. Aethyloleat oder Triglyceride, verwendet oder wässerige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffs kann von verschiedenen Faktoren, wie Applikationsweise, Warmblüter-Spezies, Alter und/oder individuellem Zustand abhängen. Im Normalfall ist für einen etwa 75 kg schweren Warmblüter bei oraler Applikation eine ungefähre Tagesdosis von etwa 20 bis etwa 500 mg, insbesondere von etwa 25 bis etwa 250 mg, vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Infolge der engen Beziehung zwischen einer Verbindung der Formel I und der entsprechenden tautomeren Verbindung der Formel I' ist in den Beispielen unter einer Verbindung der Formel I sinn- und zweckgemäss gegebenenfalls auch die tautomere Verbindung der Formel I' zu verstehen. Analoges gilt für eine Verbindung der Formel I' sowie für Salze von Verbindungen der Formeln I und I'.

<u>Beispiel 1:</u> 2,54 g (10 mmol) 1-Brom-2-(2,4-dichlorphenoxy)-äthan und 2,66 g (12 mmol) 1,2,5,6-Tetrahydro-pyridin-3-carbonsäuremethylester-hydrobromid werden in 20 ml absolutem Dimethylformamid gelöst. Dann wird bei Raumtemperatur eine Lösung von 2,84 g (22 mmol) N-Aethyl-N,N-diisopropyl-amin in 20 ml absolutem Toluol zugetropft. Das Gemisch bleibt 48 Std. bei Raumtemperatur stehen und wird anschliessend 12 Std. bei 50° gerührt. Zur Aufarbeitung werden die Lösungsmittel im Vakuum bzw. Hochvakuum bei höchstens 40-50° weitgehend entfernt und der erhaltene Rückstand in 30 ml 2N-Salzsäure gelöst. Die saure wässrige Lösung wird mit Diäthyläther ausgeschüttelt, die wässrige Phase abgetrennt und die organische Phase mit Wasser nachgewaschen. Die vereinigten wässrigen Extrakte werden kalt mit gesättigter Natriumhydrogencarbonatlösung basisch gestellt und viermal mit Diäthyläther/Dichlormethan (3:1) ausgeschüttelt. Die organischen Phasen werden mit gesättigter Natriumchloridlösung gewaschen, über Magnesiumsulfat getrocknet und im Vakuum zur Trockne eingedampft. Das erhaltene Oel, bestehend aus rohem 1-[2-(2,4-Di chlorphenoxy)äthyl]-1,2,5,6-tetrahydro-pyridin-3-carbonsäuremethylester, wird aus Diäthyläther/Pentan kristallisiert und ergibt die reine Verbindung vom Smp. 63-66°.

30 g (9.08 mmol) reiner 1-[2-(2,4-Dichlorphenoxy)-äthyl]-1,2,5,6-tetrahydro-pyridin-3-carbonsäuremethylester werden in wenig Isopropanol gelöst und bis zur stark sauren Reaktion mit ätherischer Salzsäure versetzt. Das ausgefallene Produkt wird abfiltriert und aus Isopropanol/Methanol/Diäthyläther umkristallisiert.

Das reine 1-[2-(2,4-Dichlorphenoxy)äthyl]-1,2,5,6-tetrahydro-pyridin-3-carbonsäuremethylester-hydrochlorid (Ausbeute: 90 % d.Th.) schmilzt bei 172-175°.

Beispiel 2: Analog Beispiel 1 wird aus 2,31 g (10 mmol) 1-Brom-2-(o-methoxyphenoxy)-äthan, 2,66 g (12 mmol) 1,2,5,6-Tetrahydropyridin-3-carbonsäuremethylester-hydrobromid und 2,84 g (22 mmol) N-Aethyl-N,N-diisopropyl-amin der rohe 1-[2-(o-Methoxyphenoxy)äthyl]-1,2,5,6-tetrahydro-pyridin-3-carbonsäuremethylester erhalten. Die rohe Base wird mit Cyclohexan an Florisil gereinigt und anschliessend in Isopropanol/Diäthyläther in das Oxalat überführt (Ausbeute: 77 % d.Th.). Das reine 1-[2-(o-Methoxyphenoxy)äthyl]-1,2,5,6-tetrahydro-pyridin-3-carbonsäuremethylester-oxalat schmilzt bei 128-131°.

Beispiel 3: Analog Beispiel 1 wird aus 2,31 g (10 mmol) 1-Brom-2-(m-methoxyphenoxy)-äthan, 2,66 g (12 mmol) 1,2,5,6-Tetrahydropyridin-3-carbonsäuremethylester-hydrobromid und 2,84 g (22 mmol) N-Aethyl-N,N-diisopropyl-amin der 1-[2-(m-Methoxyphenoxy)äthyl]-1,2,5,6-tetrahydro-pyridin-3-carbonsäure-methylester erhalten. Aus der freien Base wird mit ätherischer Salzsäure das Hydrochlorid gefällt und aus Isopropanol/Diäthyläther umkristallisiert. Das reine 1-[2-(m-Methoxyphenoxy)äthyl]-1,2,5,6-tetrahydro-pyridin-3-carbonsäuremethylester-hydrochlorid fällt in einer Ausbeute von 82 % d. Th. an und schmilzt bei 155-157°.

Beispiel 4: Analog Beispiel 1 wird aus 2,35 g (10 mmol) 1-Brom-2-(p-chlorphenoxy)-äthan, 2,66 g (12 mmol) 1,2,5,6-Tetrahydro-pyridin-3-carbonsäuremethylester-hydrobromid und 2,84 g (22 mmol) N-Aethyl-N,N-diisopropyl-amin der 1-[2-(p-Chlorphenoxy)äthyl]-1,2,5,6-tetrahydro-pyridin-3-carbonsäuremethylester erhalten. Aus der freien Base wird mit ätherischer Salzsäure das Hydrochlorid gefällt und dieses aus Isopropanol/Diäthyläther umkristallisiert. Das reine 1-[2-(p-Chlorphenoxy)äthyl]-1,2,5,6-tetrahydro-pyridin-3-carbonsäuremethylester-hydrochlorid schmilzt bei 158-160°. (Ausbeute: 74 % d.Th.).

Beispiel 5: Analog Beispiel 1 wird aus 5,78 g (25 mmol) 1-Brom-2-(p-methoxyphenoxy)-äthan, 6,66 g (30 mmol) 1,2,5,6-Tetrahydro-pyridin-3-carbonsäuremethylester-hydrobromid und 7,10 g (55 mmol) N-Aethyl-N,N-diisopropyl-amin der 1-[2-(p-Methoxyphenoxy)äthyl]-1,2,5,6-tetrahydro-pyridin-3-carbonsäuremethylester erhalten. Die freie Base wird mit ätherischer Salzsäure in das Hydrochlorid überführt und dieses aus Isopropanol/Diäthyläther unkristallisiert. Das reine 1-[2-(p-Methoxyphenoxy)äthyl]-1,2,5,6-tetrahydro-pyridin-3-carbonsäuremethylester-hydrochlorid (Ausbeute: 77 % d.Th.), das 0,25 Aequivalente Kristallwasser einschliesst, schmilzt bei 131-134°.

Beispiel 6: Analog Beispiel 1 wird aus 5,65 g (25 mmol) 1-Brom-2-(p-cyanophenoxy)-äthan, 6,66 g (30 mmol) 1,2,5,6-Tetrahydro-pyridin-3-carbonsäuremethylester-hydrobromid und 7,10 g (55 mmol) N-Aethyl-N,N-diisopropyl-amin der rohe 1-[2-(p-Cyanophenoxy)äthyl]-1,2,5,6-tetrahydro-pyridin-3-carbonsäuremethylester erhalten. Die Rohbase wird aus Toluol/Hexan umkristallisiert und ergibt den reinen 1-[2-(p-Cyanophenoxy)äthyl]-1,2,5,6-tetrahydro-pyridin-3-carbonsäuremethylester in einer Ausbeute von 80 % d.Th. (Smp. 81-83°).

Beispiel 7: Analog Beispiel 1 wird aus 13,5 g (50 mmol) 1-Brom-2-(m-trifluormethylphenoxy)-äthan, 13,3 g (60 mmol) 1,2,5,6-Tetrahydro-pyridin-3-carbonsäuremethylester-hydrobromid und 14,2 g (110 mmol) N-Aethyl-N,N-diisopropyl-amin der rohe 1-[2-(m-Trifluormethylphenoxy)äthyl]-1,2,5,6-tetrahydro-pyridin-3-carbonsäuremethylester erhalten. Das Rohprodukt wird mit Cyclohexan an 12 g Florisil gereinigt. Danach wird mit ätherischer Salzsäure aus der gereinigten Base das Hydrochlorid hergestellt und aus Isopropanol/Diäthyläther unkristallisiert. Man erhält das reine 1-[2-(m-Trifluormethylphenoxy)äthyl]-1,2,5,6-tetrahydro-pyridin-3-carbonsäuremethylester-hydrochlorid in einer Ausbeute von 60 % d.Th. (Smp. 144-147°).

Beispiel 8: Analog Beispiel 1 wird aus 6,45 g (30 mmol) 1-Brom-2-(p-methylphenoxy)-äthan, 8,0 g (36 mmol) 1,2,5,6-Tetrahydro-pyridin-3-carbonsäuremethylester-hydrobromid und 8,53 g (66 mmol) N-Aethyl-N,N-diisopropyl-amin der rohe 1-[2-(p-Methylphenoxy)äthyl]-1,2,5,6-tetrahydro-pyridin-3-carbonsäuremethylester erhalten. Aus der Rohbase wird mit ätherischer Salzsäure das Hydrochlorid hergestellt und dieses aus Isopropanol/Diäthyläther umkristallisiert. Das reine 1-[2-(p-Methylphenoxy)äthyl]-1,2,5,6-tetrahydro-pyridin-3-carbonsäuremethylester-hydrochlorid, das mit 0,25 Aequivalenten Kristallwasser kristallisiert, schmilzt bei 140-143° (Ausbeute: 73 % d.Th.).

Beispiel 9: 4,8 g (14 mmol) N,N-Bis(2-methoxycarbonyläthyl)-N-[2-(p-chlorphenoxy)äthyl]-amin werden in 50 ml absolutem Toluol gelöst und bei 40-50° zu einer Suspension von 0,8 g (16,8 mmol) Natriumhydrid (50 %-Suspension in Mineralöl) in 50 ml Toluol getropft. Das Reaktionsgemisch wird 3 Std. auf 80° erwärmt. Anschliessend wird bei einer Innentemperatur von 110° ein Gemisch von Toluol und Methanol abdestilliert. Der erhaltene Rückstand wird auf ein Eis/Salzsäure-Gemisch gegossen. Die organische Phase wird abgetrennt und die saure wässrige Phase mit gesättigter Natriumhydrogencarbonatlösung alkalisch gestellt. Das Gemisch wird viermal mit Diäthyläther/Dichlormethan (3:1) ausgeschüttelt und die organischen Phasen werden mit gesättigter Natriumchloridlösung gewaschen und über Magnesiumsulfat getrocknet. Die Lösungsmittel werden im Vakuum entfernt; der erhaltene ölige Rückstand wird mit Cyclohexan an 5 g Florisil gereinigt. Das erhaltene Eluat wird zur Trockne eingedampft und der Rückstand mit ätherischer Salzsäure versetzt. Das ausgefallene Hydrochlorid wird aus wenig Isopropanol/Diäthyläther umkristallisiert. Das reine 1-[2-(p-Chlorphenoxy)äthyl]-4-hydroxy-1,2,5,6-tetrahydro-pyridin-3-carbonsäuremethylester-hydrochlorid bzw. das 1-[2-(p-Chlorphenoxy)äthyl]-4-oxo-piperidin-3-carbonsäuremethylester-hydrochlorid schmilzt bei 163-164° (Ausbeute: 71 % d. Th.).

N,N-Bis(2-methoxycarbonyläthyl)-N-[2-(p-chlorphenoxy)äthyl]-amin kann z.B. wie folgt erhalten werden:

17,1 g (100 mmol) 2-(p-Chlorphenoxy)äthylamin und 21,5 g (250 mmol) Acrylsäuremethylester werden in 200 ml Methanol gelöst und 12 Std. unter Rückfluss gekocht. Das Reaktionsgemisch wird anschliessend bei

vermindertem Druck zur Trockne eingedampft. Es wird das rohe, ölige N,N-Bis(2-methoxycarbonyläthyl)-N-[2-(p-chlorphenoxy)äthyl]-amin weiter verwendet (Ausbeute: 100 % d.Th.).

Beispiel 10: Aus 6,79 g (20 mmol) N,N-Bis(2-methoxycarbonyläthyl)-N-[2-(p-methoxyphenoxy)äthyl]-amin und 1,15 g (24 mmol) Natriumhydriddispersion (50 % in Mineralöl) wird analog Beispiel 9 das 1-[2-(p-Methoxyphenoxy)äthyl]-4-hydroxy-1,2,5,6-tetrahydro-pyridin-3-carbonsäuremethylester-hydrochlorid bzw. das 1-[2-(p-Methoxyphenoxy)äthyl]-4-oxo-piperidin-3-carbonsäuremethylester-hydrochlorid in einer Ausbeute von 60 % d.Th. erhalten (Smp. 170-172°).

N,N-Bis(2-methoxycarbonyläthyl)-N-[2-(p-methoxyphenoxy)äthyl]-amin kann man z.B. auf folgende Weise herstellen:

8,06 g (50 mmol) 2-(p-Methoxyphenoxy)äthylamin und 12,9 g (150 mmol) Acrylsäuremethylester werden in 100 ml Methanol gelöst und 12 Std. unter Rückfluss gekocht. Nach Entfernen des Lösungsmittels bei vermindertem Druck wird das rohe N,N-Bis(2-methoxycarbonyläthyl)-N-[2-(p-methoxyphenoxy)äthyl]-amin als viskoses Oel in einer Ausbeute von 100 % d.Th. erhalten.

Beispiel 11: Aus 10,81 g (30 mmol) N,N-Bis-(2-methoxycarbonyläthyl)-N-[2-(o-methoxyphenoxy)äthyl]-amin und 1,73 g (36 mmol) Natriumhydriddispersion (50 % in Mineralöl) wird analog Beispiel 9 das 1-[2-(o-Methoxyphenoxy)äthyl]-4-hydroxy-1,2,5,6-tetrahydropyridin-3-carbonsäuremethylester-hydrochlorid bzw. das 1-[2-(o-Methoxyphenoxy)äthyl]-4-oxo-piperidin-3-carbonsäuremethylesterhydrochlorid in einer Ausbeute von 60 % d.Th. erhalten (Smp. 138-140°).

N,N-Bis(2-methoxycarbonyläthyl)-N-[2-(o-methoxyphenoxy)äthyl]-amin kann z.B. folgendermassen hergestellt werden:

8,06 g (50 mmol) 2-(o-Methoxyphenoxy)äthylamin und 12,9 g (150 mmol) Acrylsäuremethylester werden in 100 ml Methanol gelöst und 12 Std. unter Rückfluss gekocht. Nach Entfernen des Lösungsmittels bei vermindertem Druck wird das rohe N,N-Bis(2-methoxycarbonyläthyl)-N-[2-(o-methoxyphenoxy)äthyl]-amin in Form eines Oels in 100 % Ausbeute erhalten.

Beispiel 12: Aus 7,82 g (20 mmol) N,N-Bis(2-methoxycarbonyläthyl)-N-[3-(m-trifluormethylphenoxy)propyl]-amin und 1,15 g (24 mmol) Natriumhydriddispersion (50 % in Mineralöl) wird analog Beispiel 9 das 4-Hydroxy-1-[3-(m-trifluormethylphenoxy)propyl]-1,2,5,6-tetrahydro-pyridin-3-carbonsäuremethylester-hydrochlorid bzw. das 4-Oxo-1-[3-(m-trifluormethylphenoxy)propyl]-piperidin-3-carbonsäuremethylester-hydrochlorid erhalten.

N,N-Bis(2-methoxycarbonyläthyl)-N-[3-(m-trifluormethylphenoxy)-propyl]-amin lässt sich z.B. wie folgt herstellen:

39,1 g (100 mmol) 1-Brom-3-(m-trifluormethylphenoxy)-propan und 7,8 g (120 mmol) Natriumazid werden in 50 ml absolutem Dimethylsulfoxid gelöst und 12 Std. bei 80-100° gerührt. Das Reaktionsgemisch wird danach auf 200 ml Eiswasser gegossen. Man schüttelt mit Diäthyläther aus und wäscht die ätherischen Phasen mit gesättigter Natriumchloridlösung. Die organischen Phasen werden über Magnesium sulfat getrocknet und das Lösungsmittel wird im Vakuum entfernt. Der erhaltene ölige Rückstand, bestehend aus rohem 1-Azido-3-(m-trifluormethylphenoxy)-propan, wird ohne zusätzliche Reinigung weiterverwendet.

20 g (81,5 mmol) 1-Azido-3-(m-trifluormethylphenoxy)-propan (Rohprodukt) werden in 100 ml absolutem Diäthyläther gelöst und bei Rückflusstemperatur zu einer Suspension von 1,5 g (40 mmol) Lithiumaluminumhydrid in 150 ml absolutem Diäthyläther getropft. Das Reaktionsgemisch wird 6 Std. unter Rückfluss gekocht und anschliessend unter Eiskühlung mit 7,5 ml 0,5N-Natronlauge zersetzt. Der entstandene weisse Niederschlag wird abfiltriert und zweimal mit Diäthyläther ausgekocht. Die vereinigten ätherischen Lösungen werden über Magnesiumsulfat getrocknet und im Vakuum zur Trockne eingedampft. Das erhaltene Oel ist das 3-(m-Trifluormethylphenoxy)-propylamin, welches gegebenenfalls über sein Hydrochlorid gereinigt werden kann.

15 g (68 mmol) 3-(m-Trifluormethylphenoxy)propylamin und 14,6 g (170 mmol) Acrylsäuremethylester werden in 150 ml Methanol gelöst und 12 Std. unter Rückfluss gekocht. Nach Entfernen des Lösungsmittels unter vermindertem Druck erhält man das rohe N,N-Bis(2-methoxycarbonyläthyl)-N-[3-(m-trifluormethylphenoxy)propyl]-amin, das ohne zusätzliche Reinigung weiterverwendet werden kann.

Beispiel 13: In analoger Weise wie in Beispiel 9 beschrieben erhält man aus 6,47 g (20 mmol) N,N-Bis(2-methoxycarbonyläthyl)-N-[2-(p-methylphenoxy)äthyl]-amin und 1,15 g (24 mmol) Natriumhydrid (50 %-Suspension in Mineralöl) das 4-Hydroxy-1-[2-(p-methylphenoxy)äthyl]-1,2,5,6-tetrahydro-pyridin-3-carbonsäuremethylester-hydrochlorid bzw. das 1-[2-(p-Methylphenoxy)äthyl]-4-oxo-piperidin-3-carbonsäuremethylester-hydrochlorid in einer Ausbeute von 80 % d.Th. (Smp. 168-169°).

N,N-Bis(2-methoxycarbonyläthyl)-N-[2-(p-methylphenoxy)äthyl]-amin dann z.B. wie folgt erhalten werden

15,1 g (100 mmol) 2-(p-Methylphenoxy)äthyl-amin werden in 200 ml Methanol gelöst und mit 21,8 g (250 mmol) Acrylsäuremethylester versetzt. Das Gemisch wird 12 Std. unter Rückfluss gekocht und anschliessend nach dem Abkühlen bei vermindertem Druck zur Trockne eingedampft. Es wird das rohe, ölige N,N-Bis(2-methoxycarbonyläthyl)-N-[2-(p-methylphenoxy)äthyl]-amin weiter verwendet (Ausbeute: 90 % d.Th.).

Beispiel 14: 6,32 g (20 mmol) 4-Hydroxy-1-[2-(p-methylphenoxy)äthyl]-1,2,5,6-tetrahydro-pyridin-3-carbonsäuremethylester-hydrochlorid bzw. 1-[2-(p-Methylphenoxy)äthyl]-4-oxo-piperidin-3-carbonsäuremethylester-hydrochlorid werden in 100 ml Methanol gelöst. Bei einer Temperatur von -20 bis -10° werden 1,5 g (40 mmol) Natriumborhydrid portionsweise eingetragen. Das Reaktionsgemisch wird sodann 1 Stunde ohne Kühlung gerührt, wobei seine Temperatur auf 0° ansteigt. Das Reaktionsgemisch wird danach unter erneuter

Kühlung bis zur stark sauren Reaktion mit 2N-Salzsäure versetzt und anschliessend unter vermindertem Druck zur Trockne eingedampft. Der Rückstand wird in Eiswasser gelöst und die Lösung bis zur alkalischen Reaktion mit gesättigter Kaliumcarbonatlösung versetzt und mit Essigester ausgeschüttelt. Die vereinigten organischen Phasen werden mit gesättigter Natriumchloridlösung neutral gewaschen, über Magnesiumsulfat getrocknet und zur Trockne eingedampft. Der in Form eines viskosen Oeles erhaltene 4-Hydroxy-1-[2-(p-methylphenoxy)äthyl]-piperidin-3-carbonsäuremethylester (Ausbeute: 90 % d.Th.; cis- und trans-Diastereomerengemisch) wird ohne zusätzliche Reinigung weiter verwendet. Dazu werden 5,28 g (18 mmol) dieses Produktes und 13,7 g (90 mmol) 1,5-Diaza-bicyclo[5,4,0]undec-5-en in 90 ml Toluol gelöst. Bei 0° wird unter Rühren eine Lösung von 2,47 g (21,6 mmol) Methansulfonsäurechlorid in 15 ml Toluol innerhalb von 30 Minuten zugetropft. Das Reaktionsgemisch wird anschliessend 18 Std. bei Raumtemperatur gerührt und sodann mit 100 ml Eiswasser und 50 ml 2N-Salzsäure versetzt. Die saure wässrige Phase wird abgetrennt und die Toluolphase zweimal mit je 50 ml 1N-Salzsäure ausgeschüttelt. Die vereinigten sauren wässrigen Phasen werden mit festem Natriumhydro gencarbonat bis zur alkalischen Reaktion versetzt und dann mit Dichlormethan ausgeschüttelt. Die Dichlormethan-Extrakte werden mit gesättigter Natriumchloridlösung gewaschen und über Magnesiumsulfat getrocknet. Nach Entfernung des Lösungsmittels wird ein viskoses Oel als Rückstand erhalten. Dieses Oel wird in wenig Isopropanol gelöst. Die Lösung wird mit ätherischer Salzsäure versetzt und die ausgefallenen Kristalle werden abfiltriert. Das reine 1-[2-(p-Methylphenoxy)äthyl]-1,2,5,6-tetrehydro-pyridin-3-carbonsäuremethylester-hydrochlorid, das mit 0,25 Aequivalenten Kristallwasser kristallisiert, schmilzt bei 140-143° (Ausbeute: 70 % d.Th.).

Beispiel 15: 3,16 g (10 mmol) 1-[2-(p-Methylphenoxy)äthyl]-1,2,5,6-tetrahydro-pyridin-3-carbonsäuremethylester-hydrochlorid, welches 0,25 Aequivalente Kristallwasser enthält, werden In 50 ml absolutem Methanol gelöst und unter Zusatz von 1,0 g Palladium auf Aktivkohle unter Normaldruck bei Raumtemperatur hydriert. Der Katalysator wird abfiltriert und das Filtrat zur Trockne eingedampft. Der erhaltene Rückstand wird in 50 ml Wasser gelöst und die Lösung mit gesättigter Natriumhydrogencarbonatlösung bis zur alkalischen Reaktion versetzt und mit Dichlormethan ausgeschüttelt. Die Dichlormethan-Phasen werden mit gesättigter Natriumchloridlösung gewaschen, über Magnesiumsulfat getrocknet und unter vermindertem Druck zur Trockne eingedampft. Der als Rückstand hinterbleibende ölige rohe 1-[2-(p-Methylphenoxy)äthyl]-piperidin-3-carbonsäuremethylester wird mit Cyclohexan an Florisil gereinigt und anschiessend in Isopropanol/Diäthyläther in das Oxalat überführt (Ausbeute: 75 % d.Th.). Das reine 1-[2-(p-Methylphenoxy)äthyl]-piperidin-3-carbonsäuremethylesteroxalat schmilzt bei 146-148°.

Beispiel 16: In analoger Weise wie in Beispiel 1 beschrieben wird aus 5,66 g (20 mmol) 1-Brom-3-(m-trifluormethylphenoxy)-propan, 5,33 g (24 mmol) 1,2,5,6-Tetrahydro-pyridin-3-carbonsäuremethylester-hydrobromid und 5,68 g (44 mmol) N-Aethyl-N,N-diisopropyl-amin der 1-[3-(m-Trifluormethylphenoxy)propyl]-1,2,5,6-tetrahydro-pyridin-3-carbonsäuremethylester erhalten. Aus der freien Base wird mit ätherischer Salzsäure das Hydrochlorid gefällt. Nach Umkristal lisation aus Isopropanol/Diäthyläther erhält man das reine 1-[3-(m-Trifluormethylphenoxy)propyl]-1,2,5,6-tetrahydro-pyridin-3-carbon-säuremethylester-hydrochlorid in einer Ausbeute von 77 % d.Th. (Smp. 186-188°).

Beispiel 17: Aus 7,55 g (20 mmol) N,N-Bis(2-methoxycarbonyläthyl)-N-[2-(m-trifluormethylphenoxy)äthyl]-amin und 1,15 g (24 mmol) Natriumhydrid (50 %-Suspension in Mineralöl) wird in analoger Weise wie in Beispiel 9 beschrieben das 4-Hydroxy-1-[2-(m-trifluormethylphenoxy)äthyl]-1,2,5,6-tetrahydro-pyridin-3-carbonsäuremethylester-hydrochlorid bzw. das 4-Oxo-1-[2-(m-trifluormethylphenoxy)äthyl]-piperidin-3-carbonsäuremethylester-hydrochlorid erhalten, das einen Schmelzpunkt von 151-153° aufweist (Ausbeute: 53 % d.Th.).

Das N,N-Bis(2-methoxycarbonyläthyl)-N-[2-(m-trifluormethylphenoxy)-äthyl]-amin kann z.B. wie folgt erhalten werden:

20,5 g (100 mmol) 2-(m-Trifluormethylphenoxy)äthylamin werden in 200 ml Methanol gelöst und mit 21,73 g (250 mmol) Acrylsäuremethylester 24 Std. unter Rückfluss gekocht. Das Reaktionsgemisch wird nach dem Abkühlen unter vermindertem Druck zur Trockne eingedampft. Der ölige Rückstand wird an 10 g Magnesiumsilikat mit Dichlormethan als Laufmittel gereinigt. Nach Entfernen des Lösungsmittels im Vakuum wird das N,N-Bis(2-methoxycarbonyläthyl)-N-[2-(m-trifluormethylphenoxy)äthyl]-amin in einer Ausbeute von 98 % d.Th. erhalten.

Beispiel 18: In analoger Weise wie in Beispiel 1 beschrieben kann man aus 1,2,5,6-Tetrahydro-pyridin-3-carbonsäuremethylester-hydrobromid auch die folgenden Verbindungen erhalten:
Durch Umsetzung mit 1-Brom-2-(3,4-dichlorphenoxy)-äthan das 1-[2-(3,4-Dichlorphenoxy)äthyl]-1,2,5,6-tetrahydro-pyridin-3-carbonsäuremethylester-hydrochlorid vom Smp. 205-208° (aus Isopropanol/Diäthyläther);
durch Umsetzung mit 1-Brom-2-(2,5-dimethylphenoxy)-äthan das 1-[2-(2,5-Dimethylphenoxy)äthyl]-1,2,5,6-tetrahydro-pyridin-3-carbonsäuremethylester-oxalat vom Smp. 132-134° (aus Isopropanol);
durch Umsetzung mit 1-Brom-2-(m-methylphenoxy)-äthan das 1-[2-(m-Methylphenoxy)äthyl]-1,2,5,6-tetrahydro-pyridin-3-carbonsäuremethylester-hydrochlorid vom Smp. 153-155° (aus Isopropanol/Diäthyl-äther);
durch Umsetzung mit 1-Brom-2-(o-methylphenoxy)-äthan das 1-[2-(o-Methylphenoxy)äthyl]-1,2,5,6-tetrahydro-pyridin-3-carbonsäuremethylester-oxalat vom Smp. 165-167° (aus Isopropanol/Methanol) und
durch Umsetzung mit 1-Brom-2-(3,4-dimethylphenoxy)-äthan das 1-[2-(3,4-Dimethylphenoxy)äthyl]-1,2,5,6-tetrahydro-pyridin-3-carbonsäuremethylester-hydrochlorid vom Smp. 184-186° (aus Isopropanol).

Beispiel 19: 5,4 g (20 mmol) 1-Brom-2-(m-trifluormethylphenoxy)-äthan und 3,5 g (22 mmol) Piperidin-3-carbonsäureäthylester werden in 20 ml absolutem Dimethylformamid gelöst. Bei Raumtemperatur wird eine Lösung von 3,2 g (25 mmol) N-Aethyl-N,N-diisopropyl-amin in 20 ml Toluol zugetropft. Sodann wird das Reaktionsgemisch unter Rühren 2 Tage bei 50° gehalten und anschliessend im Hochvakuum bei 40-50° vom Lösungsmittel befreit. Der erhaltene ölige Rückstand wird in 20 ml 2N-Salzsäure gelöst. Die Lösung wird mit Diäthyläther ausgeschüttelt und die ätherische Phase mit Wasser gewaschen. Die sauren wässrigen Extrakte werden mit gesättigter Natriumhydrogen-carbonatlösung alkalisch gestellt und mit Diäthyläther/Dichlormethan (2:1) ausgeschüttelt. Die organische Phase wird mit gesättigter Natriumchlorid-lösung gewaschen, über Magnesiumsulfat getrocknet und unter vermindertem Druck eingedampft. Der erhaltene ölige Rückstand wird mit Cyclohexan als Laufmittel an Magnesiumsilikat gereinigt. Nach Entfernen des Lösungsmittels wird der hinterbleibende ölige 1-[2-(m-Trifluormethylphenoxy)äthyl]-piperidin-3-carbonsäreäthylester in wenig Diäthyläther gelöst und aus der Lösung mit ätherischer Oxalsäure das Oxalat gefällt. Nach Umkristallisation aus Methanol/Isopropanol weist das reine 1-[2-(m-Trifluormethylphenoxy)äthyl]-piperidin-3-carbonsäureäthylester-oxalat einen Smp. von 143-145° auf (Ausbeute: 62 % d.Th.).

Beispiel 20: In analoger Weise wie in Beispiel 19 beschrieben werden aus 5,7 g (20 mmol) 1-Brom-3-(m-trifluormethylphenoxy)-propan, 3,5 g (22 mmol) Piperidin-3-carbonsäureäthylester und 3,2 g (25 mmol) N-Aethyl-N,N-diisopropyl-amin 5,6 g 1-[3-(m-Trifluormethylphenoxy)-propyl]-piperidin-3-carbonsäureäthylester in Form eines farblosen Oeles erhalten. Die rohe Base wird in Diäthyläther gelöst und aus der Lösung mit ätherischer Salzsäure das Hydrochlorid gefällt. Nach Umkristallisation aus Isopropanol/Diäthyläther schmilzt das reine 1-[3-(m-Trifluormethylphenoxy)propyl]-piperidin-3-carbonsäureäthylester-hydrochlorid bei 155-157° (Ausbeute: 67 % d.Th.).

Beispiel 21: In analoger Weise wie in Beispiel 19 beschrieben werden aus 4,3 g (20 mmol) 1-Brom-2-(p-methylphenoxy)-äthan, 3,5 g (22 mmol) Piperidin-3-carbonsäureäthylester und 3,2 g (25 mmol) N-Aethyl-N,N-diisopropyl-amin nach 4 Tagen Stehenlassen bei Raumtemperatur 4,6 g öliger 1-[2-(p-Methylphenoxy)äthyl]-piperidin-3-carbonsäureäthylester erhalten. Die rohe Base wird in Diäthyläther gelöst und aus der Lösung mit ätherischer Oxalsäure das Oxalat gefällt. Nach Umkristallisation aus Isopropanol/Diäthyläther schmilzt das reine 1-[2-(p-Methylphenoxy)äthyl]-piperidin-3-carbonsäureäthylester-oxalat bei 114-116° (Ausbeute: 70 % d.Th.).

Beispiel 22: In analoger Weise wie in Beispiel 19 beschrieben kann man aus Piperidin-3-carbonsäureäthylester auch die folgenden Verbindungen erhalten:
Durch Umsetzung mit 1-Brom-2-(2,4-dichlorphenoxy)-äthan das 1-[2-(2,4-Dichlorphenoxy)äthyl]-piperidin-3-carbonsäureäthylester-oxalat vom Smp. 132-134° (aus Isopropanol/Diäthyläther);
durch Umsetzung mit 1-Brom-2-(3,4-dichlorphenoxy)-äthan das 1-[2-(3,4-Dichlorphenoxy)äthyl]-piperidin-3-carbonsäureäthylester-oxalat vom Smp. 142-144° (aus Methanol/Isopropanol/Diäthyläther);
durch Umsetzung mit 1-Brom-2-(2,5-dimethylphenoxy-äthan das 1-[2-(2,5-Dimethylphenoxy)äthyl]-piperidin-3-carbonsäureäthylester-oxalat vom Smp. 125-126° (aus Isopropanol/Diäthyläther);
durch Umsetzung mit 1-Brom-2-(m-methylphenoxy)-äthan das 1-[2-(m-Methylphenoxy)äthyl]-piperidin-3-carbonsäureäthylester-oxalat vom Smp. 116-118° (aus Isopropanol/Diäthyläther);
durch Umsetzung mit 1-Brom-2-(o-methylphenoxy)-äthan das 1-[2-(o-Methylphenoxy)äthyl]-piperidin-3-carbonsäureäthylester-oxalat vom Smp. 144-146° (aus Methanol/Isopropanol/Diäthyläther);
durch Umsetzung mit 1-Brom-2-(3,4-dimethylphenoxy(-äthan das 1-[2-(3,4-Dimethylphenoxy)äthyl]-piperidin-3-carbonsäureäthylester-oxalat vom Smp. 139-140° (aus Isopropanol/Diäthyläther) und
durch Umsetzung mit 1-Brom-4-(m-trifluormethylphenoxy)-butan das 1-[4-(m-Trifluormethylphenoxy)butyl]-piperidin-3-carbonsäureäthylester-hydrochlorid vom Smp. 124-126° (aus Isopropanol/Diäthyläther).

Beispiel 23: Aus 4,9 g (20 mmol) 1-Brom-2-(2-methoxy-5-methylphenoxy)-äthan, 3,45 g (22 mol) Piperidin-3-carbonsäureäthylester und 3,23 g (25 mmol) N-Aethyl-N,N-diisopropyl-amin wird in analoger Weise wie in Beispiel 19 beschrieben der 1-[2-(2-Methoxy-5-methylphenoxy)äthyl]-piperidin-3-carbonsäureäthylester in Form eines Oeles erhalten (Ausbeute: 60 % d. Th.).

Das 1-Brom-2-(2-methoxy-5-methyl-phenoxy)-äthan wird beispielsweise wie folgt hergestellt:
13,8 g (100 mmol) 2-Methoxy-5-methyl-phenol werden in 113 g (0,6 mol) 1,2-Dibromäthan gelöst und mit 20,8 g (150 mmol) gepulvertem Kaliumcarbonat versetzt. Unter Rühren wird das Reaktionsgemisch 2 Tage unter Rückfluss gekocht. Nach dem Abkühlen wird der Niederschlag abfiltriert und der Filterrückstand mit Dichlormethan gründlich ausgewaschen. Das Filtrat wird mit 100 ml 2N-Natronlauge ausgeschüttelt, mit gesättigter Natriumchloridlösung neutral gewaschen und über Magnesiumsulfat getrocknet. Nach Entfernen des Lösungsmittels unter vermindertem Druck wird das 1-Brom-2-(2-methoxy-5-methyl-phenoxy)-äthan in Form eines gelben Oeles erhalten (Ausbeute: 60 % d.Th.).

Beispiel 24: 4,37 g (15 mmol) 1-[2-(p-Methylphenoxy)äthyl]-piperidin3-carbonsäureäthylester werden in 30 ml n-Butanol gelöst und mit einer Lösung von 1,8 g (18 mmol) konzentrierter Schwefelsäure in 20 ml n-Butanol versetzt. Das Reaktionsgemisch wird 12 Std. unter Rühren bei 80° gehalten und anschliessend unter vermindertem Druck eingeengt. Der ölige Rückstand wird in 50 ml Wasser gelöst, mit gesättigter Natriumhydrogencarbonatlösung bis zur alkalischen Reaktion versetzt und mit Dichlormethan ausgeschüttelt. Die vereinigten organischen Phasen werden mit gesättigter Natriumchloridlösung ausgeschüttelt und über Magnesiumsulfat getrocknet. Nach Entfernen des Lösungsmittels unter vermindertem Druck wird das als Rückstand erhaltene Oel mit Cyclohexan als Laufmittel an 5 g Magnesiumsilikat gereinigt. Das Eluat wird unter vermindertem Druck zur Trockne eingedampft. Der ölige Rückstand wird mit ätherischer Oxalsäure versetzt.

Die ausgefallenen Kristalle werden aus Isopropanol/Diäthyläther umkristallisiert. Das so erhaltene reine 1-[2-(p-Methylphenoxy)äthyl]-piperidin-3-carbonsäure-n-butylester-oxalat schmilzt bei 103-105° (Ausbeute: 80 % d.Th.).

Beispiel 25: Aus 5,5 g (16 mmol) 1-[2-(m-Trifluormethylphenoxy)-äthyl]-piperidin-3-carbonsäureäthylester, 1,8 g (18 mmol) konzen-trierter Schwefelsäure und 50 ml n-Butanol wird in analoger Weise wie in Beispiel 24 beschrieben das 1-[2-(m-Trifluormethylphenoxy)-äthyl]-piperidin-3-carbonsäure-n-butylester-oxalat vom Smp. 115-117° erhalten (Ausbeute: 83 % d.Th.).

Beispiel 26: 4,3 g (20 mmol) 1-Brom-2-(p-methylphenoxy-äthan und 3,0 g (22 mmol) Piperidin-3-carbonsäureamid werden in 20 ml Dimethylformamid gelöst. Zu der Lösung wird eine Lösung von 3,2 g (25 mmol) N-Aethyl-N,N-diisopropyl-amin in 20 ml Toluol zugefügt. Das Gemisch wird unter Rühren 48 Std. auf 50° erwärmt und anschliessend im Hochvakuum zur Trockne eingedampft. Der Rückstand wird in 1N-Salzsäure gelöst und die Lösung mit Diäthyläther ausgeschüttelt. Die ätherische Phase wird mit Wasser gewaschen. Die vereinigten sauren wässrigen Extrakte werden mit 2N-Natronlauge alkalisch gestellt und dann mit Diäthyläther/Dichlormethan (2:1) ausgezogen, mit gesättigter Natriumchloridlösung gewaschen und über Magnesiumsulfat getrocknet. Nach Entfernen des Lösungsmittels unter vermindertem Druck wird der kristalline Rückstand aus Toluol/Petroläther umkristallisiert. Das reine 1-[2-(p-Methylphenoxy)äthyl]-piperidin-3-carbonsäureamid schmilzt bei 115-117° (Ausbeute: 62 % d.Th.).

Beispiel 27: Aus 5,4 g (20 mmol) 1-Brom-2-(m-Trifluormethylphenoxy)-äthan, 3,0 g (22 mmol) Piperidin-3-carbonsäureamid und 3,2 g (25 mmol) N-Aethyl-N,N-diisopropyl-amin wird in analoger Weise wie in Beispiel 26 beschrieben das ölige 1-[2-(m-Trifluormethylphenoxy)-äthyl]-piperidin-3-carbonsäureamid erhalten. Dieses liefert nach Umsetzung mit ätherischer Oxalsäure und Umkristallisation aus Methanol/Isopropanol das reine 1-[2-(m-Trifluormethylphenoxy)äthyl]-piperidin-3-carbonsäureamid-oxalat vom Smp. 163-165° in einer Ausbeute von 50 % d.Th.

Beispiel 28: In analoger Weise wie in den Beispielen 26 und 27 beschrieben kann man aus Piperidin-3-carbonsäureamid auch die folgenden Verbindungen erhalten:

Durch Umsetzung mit 1-Brom-2-(2,4-dichlorphenoxy))-äthan das 1-[2-(2,4-Dichlorphenoxy)äthyl]-piperidin-3-carbonsäureamid vom Smp. 83-85° (aus Cyclohexan/Petroläther);

durch Umsetzung mit 1-Brom-3-(m-trifluormethylphenoxy)-propan das 1-[3-(m-Trifluormethylphenoxy)propyl]-piperidin-3-carbonsäureamid vom Smp. 57-59° (aus Toluol/Petroläther) und

durch Umsetzung mit 1-Brom-2-(3,4-dimethylphenoxy)-äthan das 1-[2-(3,4-Dimethylphenoxy)äthyl]-piperidin-3-carbonsäureamid vom Smp. 108-110° (aus Cyclohexan/Petroläther).

Beispiel 29: 4,3 g (20 mmol) 1-Brom-2-(p-methyphenoxy)-äthan und 4,1 g (22 mmol) Piperidin-3-carbonsäure-N,N-diäthylamid werden in 20 ml Dimethylformamid gelöst und mit einer Lösung von 3,2 g (25 mmol) N-Aethyl-N,N-diisopropyl-amin in 20 ml Toluol versetzt. Das Reaktionsgemisch wird unter Rühren 3 Tage auf 50° erwärmt und anschliessend im Hochvakuum zur Trockne eingedampft. Der erhaltene Rückstand wird in 50 ml 1N-Salzsäure gelöst. Die Lösung wird mit Diäthyläther ausgeschüttelt und die ätherische Phase mit Wasser nachgewaschen. Die vereinigten sauren wässrigen Phasen werden mit 2N-Natronlauge alkalisch gestellt und mit Diäthyläther/Dichlormethan (2:1) ausgeschüttelt. Die vereinigten organischen Phasen werden mit gesättigter Natriumchloridlösung gewaschen, über Magnesiumsulfat getrocknet und unter vermindertem Druck vom Lösungsmittel befreit. Der erhaltene ölige Rückstand wird an 120 g Kieselgel (Merck 0,04-0,063 mm) mit Dichlormethan/Methanol (9:1) als Laufmittel chromatographiert. Die Fraktionen 1-5 werden vereinigt und das Lösungsmittel unter vermindertem Druck entfernt. Der ölige Rückstand wird mit ätherischer Salzsäure versetzt. Die ausgefallenen Kristalle werden abfiltriert und aus Essigsäureäthylester/Diäthyläther umkristallisiert. Das so erhaltene reine 1-[2-(p-Methylphenoxy)-äthyl]-piperidin-3-carbonsäure-N,N-diäthylamid-hydrochlorid schmilzt bei 149-151° (Ausbeute: 27 % d.Th.).

Beispiel 30: In analoger Weise wie in Beispiel 29 beschrieben kann man aus Piperidin-3-carbonsäure-N,N-diäthylamid auch die folgenden Verbindungen erhalten:

Durch Umsetzung mit 1-Brom-2-(3,4-dimethylphenoxy)-äthan das 1-[2-(3,4-Dimethylphenoxy)äthyl]-piperidin-3-carbonsäure-N,N-diäthylamid-oxalat vom Smp. 128-129°;

durch Umsetzung mit 1-Brom-2-(m-trifluormethylphenoxy)-äthan das 1-[2-(m-Trifluormethylphenoxy)äthyl]-piperidin-3-carbonsäure-N,N-diäthylamid-hydrochlorid vom Smp. 120-122° und

durch Umsetzung mit 1-Brom-2-(2,4-dichlorphenoxy)-äthan das 1-[2-(2,4-Dichlorphenoxy)äthyl]-piperidin-3-carbonsäure-N,N-diäthylamid-hydrochlorid vom Smp. 201-204°.

Beispiel 31: In analoger Weise wie in Beispiel 19 beschrieben wird aus 5,9 g (20 mmol) 1-Brom-4-(m-trifluormethylphenoxy)-butan, 2,6 g (22 mmol) 3-Hydroxymethylpiperidin und 3,2 g (25 mmol) N-Aethyl-N,N-diisopropyl-amin nach Reinigung an Florisil das ölige 1-[4-(m-Trifluormethylphenoxy)butyl]-3-hydroxymethyl-piperidin erhalten. Aus der rohen Base wird mit ätherischer Oxalsäure das Oxalat gefällt und dieses aus Isopropanol/Diäthyläther umkristallisiert. Das reine 1-[4-(m-Trifluormethylphenoxy)butyl]-3-hydroxymethyl-piperidin-oxalat schmilzt bei 128-130°.

Beispiel 32: 4,9 g (20 mmol) 1-[2-(p-Methylphenoxy)äthyl]-3-cyano-piperidin werden in 160 ml absolutem Aethanol gelöst. Bei einer Temperatur von +5° wird bis zur Sättigung Chlorwasserstoffgas in die Lösung eingeleitet. Anschliessend lässt man das Gemisch 24 Std. bei Raumtemperatur stehen. Danach wird das Lösungsmittel und das überschüssige Chlorwasserstoffgas unter vermindertem Druck entfernt. Der erhaltene kristalline Rückstand wird in 50 ml Wasser gelöst und die Lösung 2 Std. bei Raumtemperatur stehengelassen und anschliessend mit Natriumhydrogencarbonat alkalisch gestellt. Das Gemisch wird mit Diäthyläther

ausgeschüttelt und die organische Phase mit gesättigter Natriumchloridlösung gewaschen und über Magnesiumsulfat getrocknet. Nach Entfernen des Lösungsmittels unter vermindertem Druck wird das erhaltene Oel mit ätherischer Oxalsäure versetzt und das Oxalat aus Isopropanol/Diäthyläther umkristallisiert. Das reine 1-[2-(p-Methylphenoxy)äthyl]-piperidin-3-carbonsäureäthylester-oxalat schmilzt bei 114-116°.

Das 1-[2-(p-Methylphenoxy)äthyl]-3-cyano-piperidin wird z.B. wie folgt hergestellt:

11,0 g (100 mmol) 3-Cyanopiperidin, 21,5 g (100 mmol) 1-Brom-2-(p-methylphenoxy)-äthan und 16,2 g (125 mmol) N-Aethyl-N,N-diisopropyl-amin werden in 50 ml Toluol und 50 ml Dimethylformamid gelöst und 48 Std. bei Raumtemperatur stehengelassen. Anschliessend werden im Hochvakuum die Lösungsmittel bei 40-50° entfernt und der erhaltene ölige Rückstand in 2N-Salzsäure gelöst. Nach Ausschütteln mit Diäthyläther wird die saure wässrige Phase mit Natriumhydrogencarbonat alkalisch gestellt und mit Diäthyläther/Dichlormethan (2:1) ausgeschüttelt. Die organischen Phasen werden mit gesättigter Natriumchloridlösung gewaschen und über Magnesiumsulfat getrocknet. Die Lösungsmittel werden unter vermindertem Druck entfernt. Der erhaltene Rückstand wird mit Cyclohexan als Laufmittel an 30 g Florisil gereinigt. Das erhaltene 1-[2-(p-Methylphenoxy)äthyl]-3-cyano-piperidin wird roh weiterverwendet.

Beispiel 33: 4,7 g (20 mmol) 1-[2-(p-Methylphenoxy)äthyl]-4-oxo-piperidin werden in 20 ml Diäthylenglykoldimethyläther (Diglyme) gelöst und die Lösung bei 50° unter Rühren zu einem Gemisch von 3,6 g (40 mmol) Dimethylcarbonat und 1,2 g (25 mmol) Natriumhydriddispersion in Mineralöl (50 %) in 20 ml Diäthylenglykoldimethyläther (Diglyme) zugetropft. Anschliessend wird das Reaktionsgemisch 12 Std. unter Rückfluss gekocht, danach abgekühlt, mit 50 ml Toluol verdünnt und tropfenweise unter Kühlung mit 1,5 g Eisessig versetzt. Die Lösungsmittel werden sodann unter vermindertem Druck weitgehend entfernt. Der Rückstand wird in Diäthyläther gelöst und die Lösung mit 2N-Salzsäure ausgeschüttelt. Die saure wässrige Phase wird mit Natriumhydrogencarbonat alkalisch gestellt und mit Diäthyläther/Dichlormethan (2:1) ausgeschüttelt. Die organischen Phasen werden über Magnesiumsulfat getrocknet und vom Lösungsmittel befreit. Der Rückstand wird mit ätherischer Salzsäure versetzt. Das ausgefallene Hydrochlorid wird aus Isopropanol/Diäthyläther umkristallisiert. Das reine 4-Hydroxy-1-[2-p-methylphenoxy)äthyl]-1,2,5,6-tetrahydropyridin-3-carbonsäuremethylester-hydrochlorid bzw. 1-[2-(p-Methylphenoxy)äthyl]-4-oxo-piperidin-3-carbonsäuremethylester-hydrochlorid schmilzt bei 168-169°.

Das 1-[2-(p-Methylphenoxy)äthyl]-4-oxo-piperidin wird beispielsweise folgendermassen erhalten:

In analoger Weise wie in Beispiel 19 beschrieben wird aus 4,3 g (20 mmol) 1-Brom-2-(p-methylphenoxy)-äthan, 3,4 g (22 mmol) 4-Piperidon-hydrat-hydrochlorid und 5,7 g (44 mmol) N-Aethyl-N,N-diisopropyl-amin das rohe 1-[2-(p-Methylphenoxy)äthyl]-4-oxo-piperidin als orangegelbes Oel erhalten. Nach Reinigung des Rohproduktes über Florisil mit Cyclohexan als Laufmittel wird mit ätherischer Oxalsäure das Oxalat gefällt und dieses aus wenig Aethanol/Diäthyläther umkristallisiert. Das reine 1-[2-(p-Methylphenoxy)äthyl]-4-oxo-piperidin-oxalat, das mit 0,125 Aequivalenten Kristallwasser kristallisiert, schmilzt bei 157-159°.

Beispiel 34:6,2 g (20 mmol) 4-Chlor-1-[2-(p-methylphenoxy)äthyl]-piperidin-3-carbonsäuremethylester werden in 20 ml Methanol gelöst. Bei Raumtemperatur werden 40 ml (140 mmol) einer 3,5N-Lösung von Ammoniak in Methanol zugetropft. Das Gemisch wird 24 Std. bei Raumtemperatur stehengelassen. Sodann wird das Lösungsmittel unter vermindertem Druck entfernt. Der erhaltene Rückstand wird in Dichlormethan gelöst, die Lösung mit 2N-Salzsäure ausgeschüttelt und die saure wässrige Phase abgetrennt, mit Natriumhydrogencarbonat alkalisch gestellt und mit Diäthyläther/Dichlormethan (2:1) extrahiert. Die organischen Extrakte werden mit gesättigter Natriumchloridlösung gewaschen, über Magnesiumsulfat getrocknet und unter vermindertem Druck von Lösungsmittel befreit. Der erhaltene Rück stand wird an basischem Kieselgel mit Dichlormethan/Methanol (99:1) als Laufmittel chromatographiert. Die Eluate werden zur Trockne eingedampft und der Rückstand mit ätherischer Salzsäure versetzt. Man erhält so das reine 4-Amino-1-[2-(p-methylphenoxy)äthyl]-piperidin-3-carbonsäureamid-dihydrochlorid.

Den 4-Chlor-1-[2-(p-methylphenoxy)äthyl]-piperidin-3-carbonsäuremethylester kann man beispielsweise wie folgt erhalten:

16,35 g (50 mmol) 4-Hydroxy-1-[2-(p-methylphenoxy)äthyl]-1,2,5,6-tetrahydro-pyridin-3-carbonsäuremethylester-hydrochlorid bzw. 1-[2-(p-Methylphenoxy)äthyl]-4-oxo-piperidin-3-carbonsäuremethylester-hydrochlorid (Herstellung siehe Beispiel 13) werden während 1 Std. portionsweise bei -20° in eine Lösung von 5,6 g (150 mmol) Natriumborhydrid in 300 ml Methanol eingetragen. Das Gemisch wird dann 3 Std. bei -20° gerührt und anschliessend im Verlaufe von 4 Std. auf Raumtemperatur aufgetaut. Danach wird das Gemisch unter Kühlung tropfenweise mit 70 ml 1N-Salzsäure versetzt. Sodann wird das Methanol unter vermindertem Druck so weit wie möglich abgezogen. Der Rückstand wird mit Diäthyläther gewaschen und die saure wässrige Phase mit Natriumhydrogencarbonat alkalisch gestellt und mit Diäthyläther/Dichlormethan (2:1) extrahiert. Die organische Phase mird mit gesättigter Natriumchloridlösung gewaschen, über Magnesiumsulfat getrocknet und unter vermindertem Druck vom Lösungsmittel befreit. Der erhaltene Rückstand, bestehend aus rohem 4-Hydroxy-1-[2-(p-methylphenoxy)äthyl]-piperidin-3-carbonsäuremethylester, wird ohne weitere Aufarbeitung weiterverwendet.

8,8 g (30 mmol) 4-Hydroxy-1-[2-(p-methylphenoxy)äthyl]-piperidin-3-carbonsäuremethylester und 3,6 g (35 mmol) Triäthylamin werden in 100 ml Dichlormethan gelöst. Bei Raumtemperatur werden unter Rühren 3,92 g (33 mmol) Thionylchlorid zugetropft. Das Gemisch wird 4 Std. bei Raumtemperatur gerührt. Sodann wird vom ausgefallenen Triäthylaminhydrochlorid abfiltriert und das Filtrat kalt mit gesättigter Natriumhydrogencarbonatlösung ausgeschüttelt. Die organische Phase wird mit gesättigter Natriumchloridlösung gewaschen, über Magnesium sulfat getrocknet und unter vermindertem Druck vom Lösungsmittel befreit. Der erhaltene

4-Chlor-1-[2-(p-methylphenoxy)äthyl]-piperidin-3-carbonsäuremethylester wird roh weiterverwendet.

Beispiel 35: 7,0 g (20 mmol) 1-[2-(p-Methylphenoxy)äthyl]-pyridinium-3-carbonsäuremethylester-bromid werden bei -15° während 1 Std. portionsweise unter Rühren in eine Lösung von 2,3 g (60 mmol) Natriumborhydrid in 200 ml Methanol eingetragen. Das Gemisch wird 1 Std. bei -10° nachgerührt. Die Reaktionslösung wird anschliessend in 1 Std. auf Raumtemperatur aufgetaut und sodann eine weitere Stunde unter Rückfluss gekocht. Nach erfolgter Abkühlung werden 50 ml 1N-Salzsäure langsam zugetropft und danach das Methanol unter vermindertem Druck entfernt. Der Rückstand wird mit Diäthyläther ausgeschüttelt und die saure wässrige Phase unter Kühlung mit Natriumhydrogencarbonat alkalisch gestellt und mit Diäthyläther/Dichlormethan (2:1) extrahiert. Die organischen Extrakte werden mit gesättigter Natriumchloridlösung gewaschen, über Magnesiumsulfat getrocknet und unter vermindertem Druck vom Lösungsmittel befreit. Der erhaltene ölige Rückstand wird mit ätherischer Salzsäure versetzt und das ausgefallene Hydrochlorid aus Isopropanol/Diäthyläther umkristallisiert. Das so erhältliche 1-[2-(p-Methylphenoxy)äthyl-1,2,5,6-tetrahydro-pyridin-3-carbonsäuremethylester-hydro-chlorid, das mit 0,25 Aequivalenten Kristallwasser kristallisiert, schmilzt bei 140-143°.

Das 1-[2-(p-Methylphenoxy)äthyl]-pyridinium-3-carbonsäuremethylester-bromid wird beispielsweise auf folgende Weise erhalten:

6,45 g (30 mmol) 1-Brom-2-(p-methylphenoxy)-äthan und 4,47 g (30 mmol) Pyridin-3-carbonsäuremethylester werden in 50 ml absolutem Trichlormethan gelöst und 24 Std. unter Rühren auf 50° erwärmt. Das ausgefallene 1-[2-(p-Methylphenoxy)äthyl]-pyridinium-3-carbonsäuremethylester-bromid wird abfiltriert und roh weiterverwendet.

Beispiel 36: In analoger Weise wie in den Beispielen 1 bis 35 beschrieben kann man auch die folgenden Verbindungen und jeweils deren pharmazeutisch verwendbare Salze erhalten:
Den 1-[2-(p-Trifluormethylphenoxy)äthyl]-1,2,5,6-tetrahydro-pyridin-3-carbonsäuremethylester;
den 1-[2-(5-Methoxy-2-methyl-phenoxy)äthyl]-1,2,5,6-tetrahydro-pyridin-3 -carbonsäuremethylester;
den 4-Hydroxy-1-[2-(5-methoxy-2-methyl-phenoxy)äthyl]-1,2,5,6-tetrahydro-pyridin-3-carbonsäuremethylester bzw. den 1-[2-(5-Methoxy-2-methyl-phenoxy)äthyl]-4-oxo-piperidin-3-carbonsäuremethylester;
den 4-Hydroxy-1-[2-(2,5-dimethoxyphenoxy)äthyl]-1,2,5,6-tetrahydro-pyridin-3-carbonsäuremethylester bzw. den 1-[2-(2,5-Dimethoxyphenoxy)äthyl]-4-oxo-piperidin-3-carbonsäuremethylester;
den 1-[2-(2,5-Dimethoxyphenoxy)äthyl-1,2,5,6-tetrahydro-pyridin-3-carbonsäuremethylester;
das 1-[4-(m-Trifluormethylphenoxy)butyl]-piperidin-3-carbonsäuramid vom Smp. 124-128°;
das 1-[2-(p-Cyanophenoxy)äthyl]-piperidin-3-carbonsäureamid vom Smp. 152-154° und
das 1-[2-(p-Methylphenoxy)äthyl]-3-hydroxymethyl-piperidin vom Smp 149-150°.

Beispiel 37: Tabletten, enthaltend 25 mg Wirkstoff, z.B. 1-[2-(2,4-Dichlorphenoxy)-äthyl]-1,2,5,6-tetrahydro-pyridin-3-carbonsäuremethylester-hydrochlorid, können folgendermassen hergestellt werden:

| Bestandteile (für 1000 Tabletten): | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann werden der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 38: Tabletten, enthaltend 50 mg des Wirkstoffs, z.B. 1-[2-(2,4-Dichlorphenoxy)-äthyl]-1,2,5,6-tetrahydro-pyridin-3-carbonsäuremethylester-hydrochlorid, werden wie folgt hergestellt:

| Zusammensetzung (für 10 000 Tabletten): | |
|---|---|
| Wirkstoff | 500,00 g |
| Lactose | 140,80 g |
| Kartoffelstärke | 274,70 g |
| Stearinsäure | 10,00 g |
| Talk | 50,00 g |
| Magnesiumstearat | 2,50 g |
| Kolloidales Siliciumdioxid | 32,00 g |
| Aethanol | q.s. |

Ein Gemisch des Wirkstoffs, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung

zu Tabletten von je 0,1 g Gewicht, die gewünschtenfalls mit Teilkerben zur feineren Anpassung der Dosierung versehen sein können.

In analoger Weise können 100 mg Wirkstoff eingearbeitet werden.

Beispiel 39: Kapseln, enthaltend 0,025 g des Wirkstoffs, z.B. 1-[2-(2,4-Dichlorphenoxy-äthyl]-1,2,5,6-tetrahydro-pyridin-3-carbonsäuremethylester-hydrochlorid, können wie folgt hergestellt werden:

Zusammensetzung (für 1000 Kapseln):

| | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2 bis 1,5 mm. Das Granulat mischt man mit der getrockneten Maisstärke und dem Talk und füllt Portionen von 300 mg in Hartgelatinekapseln (Grösse 1) ab.

Beispiel 40: In analoger Weise wie in den Beispielen 37 bis 39 beschrieben können auch pharmazeutische Präparate, enthaltend eine andere Verbindung der Formel I oder ein Tautomeres und/oder pharmazeutisch verwendbares Salz davon, beispielsweise gemäss den Beispielen 1 bis 36, hergestellt werden.

**Patentansprüche**

1. Eine Verbindung der Formel

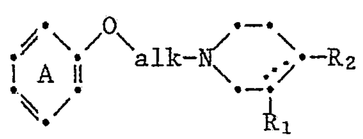

(I),

worin $R_1$ Niederalkoxycarbonyl, Carbamyl, N-Niederalkylcarbamyl, N,N-Diniederalkylcarbamyl oder gegebenenfalls acyliertes Hydroxymethyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder ein Tautomeres und/oder Salz davon.

2. Eine Verbindung der Formel I gemäss Anspurch 1, worin $R_1$ Niederalkoxycarbonyl, Carbamyl, N-Niederalkylcarbamyl oder N,N-Diniederalkylcarbamyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder ein Tautomeres und/oder Salz davon.

3. Eine Verbindung der Formel I gemäss Anspruch 1, worin $R_1$ Niederalkoxycarbonyl, Carbamyl, N-Niederalkylcarbamyl, N,N-Diniederalkylcarbamyl, Hydroxymethyl, Niederalkanoyloxymethyl, Niederalkansulfonyloxymethyl, Benzoyloxymethyl oder Pyridoyloxymethyl bedeutet, $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Niederalkansulfonylamino, Benzoylamino oder Pyridoylamino darstellt, alk Niederalkylen, das die beiden in Formel I eingezeichneten Heteroatome O und N in erster Linie durch 2 bis und mit 4 C-Atome überbrückt, bedeutet, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder ein Tautomeres und/oder Salz davon.

4. Eine Verbindung der Formel I gemäss Anspruch 1, worin $R_1$ Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Benzoylamino oder Pyridoylamino darstellt, alk Niederalkylen, das die beiden in Formel I eingezeichneten Heteroatome O und N in erster Linie durch 2 bis und mit 3 C-Atome überbrückt, bedeutet, der Ring A unsubstituiert ist oder ein-, zwei- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder

ein Tautomeres und/oder Salz davon.

5. Eine Verbindung der Formel I gemäss Anspruch 1, worin $R_1$ $C_1$ -$C_4$ -Alkoxycarbonyl, Carbamyl, N-$C_1$ -$C_4$ -Alkylcarbamyl, N,N-Di-$C_1$ -$C_4$ -Alkylcarbamyl, Hydroxymethyl oder $C_2$ -$C_5$ -Alkanoyloxymethyl bedeutet, $R_2$ Wasserstoff, Hydroxy oder Amino darstellt, alk für $C_1$ -$C_4$ -Alkylen, das die beiden in Formel I eingezeichneten Heteroatome O und N in erster Linie durch 2 bis und und mit 4 C-Atome überbrückt, steht, der Ring A unsubstituiert oder ein-, zwei- oder mehrfach durch $C_1$ -$C_4$ -Alkoxy, Cyano, Halogen mit einer Atomnummer bis und mit 35, $C_1$ -$C_4$ -Alkyl und/oder Trifluormethyl substituiert ist und die ge strickelte Linie zum Ausdruck bringen soll, dass eine Einfach- oder eine Doppelbindung vorliegt, oder ein Tautomeres und/oder Salz davon.

6. Eine Verbindung der Formel I gemäss Anspruch 1, worin $R_1$ $C_1$ -$C_4$ -Alkoxycarbonyl oder Carbamyl bedeutet, $R_2$ Wasserstoff oder Hydroxy bedeutet, alk für $C_1$ -$C_4$ -Alkylen, das die beiden in Formel I eingezeichneten Heteroatome O und N in erster Linie durch 2 bis und mit 3 C-Atome überbrückt, steht, der Ring A unsubstituiert oder ein-, zwei- oder mehrfach durch $C_1$ -$C_4$ -Alkoxy, Cyano, Halogen mit einer Atomnummer bis und mit 35, $C_1$ -$C_4$ -Alkyl und/oder Trifluormethyl substituiert ist und die gestrichelte Linie zum Ausdruck bringen soll, dass eine Doppelbindung vorliegt, oder ein Tautomeres und/oder Salz davon.

7. Eine Verbindung der Formel I gemäss Anspruch 1, worin $R_1$ $C_1$ -$C_4$ -Alkoxycarbonyl oder Carbamyl bedeutet, $R_2$ Wasserstoff oder Hydroxy darstellt, alk für $C_1$ -$C_4$ -Alkylen, das die beiden in Formel I eingezeichneten Heteroatome O und N in erster Linie durch 2 bis und mit 3 C-Atome überbrückt, steht, der Ring A einfach durch $C_1$ -$C_4$ -Alkoxy, $C_1$ -$C_4$ -Alkyl oder Trifluormethyl oder zweifach durch Halogen mit einer Atomnummer bis und mit 35 oder $C_1$ -$C_4$ -Alkyl oder $C_1$ -$C_4$ -Alkoxy sowie $C_1$ -$C_4$ -Alkyl substituiert ist und die gestrichelte Linie zum Ausdruck bringen soll, dass eine Einfach- oder eine Doppelbindung vorliegt, oder ein Tautomeres und/oder Salz davon.

8. 1-[2-(2,4-Dichlorphenoxy)äthyl]-1,2,5,6-tetrahydro-pyridin-3-carbonsäuremethylester, 1-[2-(o-Methoxyphenoxy)äthyl]-1,2,5,6-tetrahydro-pyridin-3-carbonsäuremethylester, 1-[2-(m-Trifluormethylphenoxy)äthyl]-1,2,5,6-tetrahydro-pyridin-3-carbonsäuremethylester und 1-[2-(p-Methylphenoxy)äthyl]-1,2,5,6-tetrahydro-pyridin-3-carbonsäuremethylester oder jeweils ein Salz davon.

9. 1-[2-(m-Methoxyphenoxy)äthyl]-1,2,5,6-tetrahydro-pyridin-3-carbonsäuremethylester, 1-[2-(p-Chlorphenoxy)äthyl]-1,2,5,6-tetrahydro-pyridin-3-carbonsäuremethylester, 1-[2-(p-Methoxyphenoxy)- äthyl]-1,2,5,6-tetrahydro-pyridin-3-carbonsäuremethylester, 1-[2-(p-Cyanophenoxy)äthyl]-1,2,5,6-tetrahydro-pyridin-3-carbonsäuremethylester, 1-[2-(p-Chlorphenoxy)äthyl]-4-hydroxy-1,2,5,6-tetrahydro-pyridin-3-carbonsäuremethylester bzw. 1-[2-(p-Chlorphenoxy)äthyl]-4-oxo-piperidin-3-carbonsäuremethylester, 1-[2-(p-Methoxyphenoxy)äthyl]-4-hydroxy-1,2,5,6-tetrahydro-pyridin-3-carbonsäuremethylester bzw. 1-[2-(p-Methoxyphenoxy)-äthyl]-4-oxo-piperidin-3-carbonsäuremethylester, 1-[2-(o-Methoxyphenoxy)äthyl]-4-hydroxy-1,2,5,6-tetrahydro-pyridin-3-carbonsäuremethylester bzw. 1-[2-(o-Methoxyphenoxy)-äthyl]-4-oxo-piperidin-3-carbonsäuremethylester und 4-Hydroxy-1-[3-(m-trifluormethylphenoxy)propyl]-1,2,5,6-tetrahydro-pyridin-3-carbonsäuremethylester bzw. 4-Oxo-1-[3-(m-trifluormethylphenoxy)propyl]-piperidin-3-carbonsäuremethylester oder jeweils ein Salz davon.

10. 4-Hydroxy-1-[2-(p-methylphenoxy)äthyl]-1,2,5,6-tetrahydro-pyridin-3-carbonsäuremethylester bzw. 1-[2-(p-Methylphenoxy)äthyl]-4-oxo-piperidin-3-carbonsäuremethylester, 4-Hydroxy-1-[2-(m-trifluormethylphenoxy)äthyl]-1,2,5,6-tetrahydro-pyridin-3-carbonsäuremethylester bzw. 4-Oxo-1-[2-(m-trifluormethylphenoxy)äthyl]-piperidin-3-carbonsäuremethylester, 1-[2-(m-Trifluormethylphenoxy)äthyl]-piperidin-3-carbonsäureäthylester, 1-[3-(m-Trifluormethylphenoxy)propyl]-piperidin-3-carbonsäureäthylester, 1-[2-(p-Methylphenoxy)äthyl]-piperidin-3-carbonsäureäthylester, 1-[2-(2-Methoxy-5-methyl-phenoxy)äthyl]-piperidin-3-carbonsäureäthylester, 1-[2-(3,4-Dichlorphenoxy)äthyl]-piperidin-3-carbonsäureäthylester, 1-[2-(3,4-Dimethylphenoxy)äthyl]-piperidin-3-carbonsäureäthylester und 1-[2-(p-Methylphenoxy)äthyl]-piperidin-3-carbonsäureamid oder jeweils ein Salz davon.

11. 1-[2-(p-Methylphenoxy)äthyl]-piperidin-3-carbonsäuremethylester, 1-[3-(m-Trifluormethylphenoxy)propyl]-1,2,5,6-tetrahydro-pyridin-3-carbonsäuremethylester, 1-[2-(2,4-Dichlorphenoxy)äthyl]-piperidin-3-carbonsäureäthylester, 1-[2-(3,4-Dichlorphenoxy)äthyl]-1,2,5,6-tetrahydro-pyridin-3-carbonsäuremethylester, 1-[2-(2,5-Dimethylphenoxy)äthyl]-piperidin-3-carbonsäureäthylester, 1-[2-(2,5- Dimethylphenoxy)äthyl]-1,2,5,6-tetrahydro-pyridin-3-carbonsäuremethylester, 1-[2-(m-Methylphenoxy)äthyl]-piperidin-3-carbonsäureäthylester, 1-[2-(m-Methylphenoxy)äthyl]-1,2,5,6-tetrahydro-pyridin-3-carbonsäuremethylester, 1-[2-(o-Methylphenoxy)äthyl]-piperidin-3-carbonsäureäthylester, 1-[2-(o-Methylphenoxy)äthyl]-1,2,5,6-tetrahydro-pyridin-3-carbonsäuremethylester, 1-[2-(3,4-Dimethylphenoxy)äthyl]-1,2,5,6-tetrahydro-pyridin-3-carbonsäuremethylester, 1'[2-(p-Methylphenoxy)äthyl]-piperidin-3-carbonsäure-n-butylester, 1-[2-(m-Trifluormethylphenoxy)äthyl]-piperidin-3-carbonsäureamid, 1-[2-(m-Trifluormethylphenoxy)äthyl]-piperidin-3-carbonsäure-n-butylester, 1-[2-(2,4-Dichlorphenoxy)äthyl]-piperidin-3-carbonsäureamid, 1-[3-(m-Trifluormethylphenoxy)propyl]-piperidin-3-carbonsäureamid, 1-[2-(3,4-Dimethylphenoxy)äthyl]-piperidin-3-carbonsäureamid, 1-[4-(m-Trifluormethylphenoxy)butyl]-piperidin-3-carbonsäureäthylester, 1-[2-(3,4-Dimethylphenoxy)äthyl]-piperidin-3-carbonsäure-N,N-diäthylamid, 1-[2-(m-Trifluormethylphenoxy)äthyl]-piperidin-3-carbonsäure-N,N-diäthylamid, 1-[2-(p-Methylphenoxy)äthyl]-piperidin-3-carbonsäure-N,N-diäthylamid, 1-[2-(2,4-Dichlorphenoxy)äthyl]-piperidin-3-carbonsäure-N,N-diäthylamid, 1-[4-(m-Trifluormethylphenoxy)butyl]-3-hydroxymethyl-piperidin, 4-Amino-1-(p-methylphenoxy)äthyl]-piperidin-3-carbonsäureamid, 1-[2-(p-Trifluorme-

thylphenoxy)äthyl]-1,2,5,6-tetrahydro-pyridin-3-carbonsäuremethylester, 1-[2-(5-Methoxy-2-methyl-phenoxy)äthyl]-1,2,5,6-tetrahydro-pyridin-3-carbonsäuremethylester, 4-Hydroxy-1-[2-(5-methoxy-2-methyl-phenoxy)äthyl]-1,2,5,6-tetrahydro-pyridin-3-carbonsäuremethylester bzw. 1-[2-(5-Methoxy-2-methyl-phenoxy)äthyl]-4-oxo-piperidin-3-carbonsäuremethylester, 4-Hydroxy-1-[2-(2,5-dimethoxyphenoxy)äthyl]-1,2,5,6-tetrahydro-pyridin-3-carbonsäuremethylester bzw. 1-[2-(2,5-Dimethoxyphenoxy)-äthyl]-4-oxo-piperidin-3-carbonsäuremethylester, 1-[2-(2,5-Dimethoxyphenoxy)äthyl]-1,2,5,6-tetrahydro-pyridin-3-carbonsäuremethylester, 1-[4-(m-Trifluormethylphenoxy)butyl]-piperidin-3-carbonsäureamid, 1-[2-(p-Cyanophenoxy)äthyl]-piperidin-3-carbonsäureamid und 1-[2-(p-Methylphenoxy)äthyl]-3-hydroxymethyl-piperidin oder jeweils ein Salz davon.

12. Verfahren zur Herstellung einer Verbindung der Formel I, eines Tautomeren und/oder Salzes davon gemäss einem der Ansprüche 1 bis 11, dadurch gekennzeichnet, dass man

a) eine Verbindung der Formel

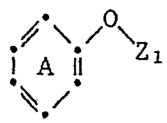

(IIa)

oder ein Salz davon mit einer Verbindung der Formel

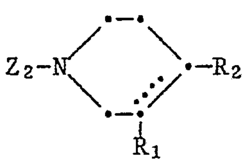

(IIb),

einem Tautomeren und/oder Salz davon, wobei entweder $Z_1$ eine Gruppe alk-$X_1$ und $Z_2$ Wasserstoff ist, oder $Z_1$ Wasserstoff und $Z_2$ eine Gruppe alk-$X_1$ darstellt, jeweils mit $X_1$ als Hydroxy oder reaktionsfähigem veresterten Hydroxy, umsetzt oder

b) in einer Verbindung der Formel

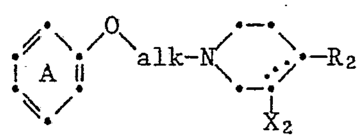

(III) ,

einem Tautomeren und/oder Salz davon, worin $X_2$ einen in $R_1$ überführbaren Rest bedeutet, $X_2$ in $R_1$ überführt oder

c) zur Herstellung einer Verbindung der Formel I, eines Tautomeren und/oder Salzes davon, worin $R_2$ Hydroxy oder Amino bedeutet, und worin $R_1$ insbesondere Niederalkoxycarbonyl bedeutet, eine Verbindung der Formel

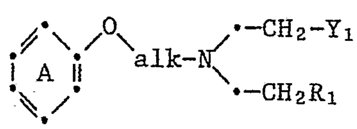

(IV),

worin $Y_1$ eine Gruppe der Formel $-CH=R_2'$, $-C(Y_2)=R_2'$, $-CH(Y_2)-R_2'$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', eines Tautomeren und/oder Salzes davon, worin $R_1$ Oxo oder Imino bedeutet und die gestrichelte Linie zum Ausdruck bringen soll, dass eine Einfachbindung vorliegt, und worin $R_1$ insbesondere Niederalkoxycarbonyl bedeutet, eine Verbindung der Formel

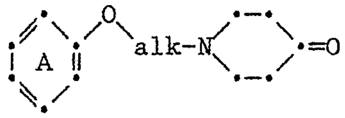

(Va)

oder ein Tautomeres oder jeweils ein Salz davon mit einer Verbindung der Formel

$X_3$ -$R_1$ (Vb)

oder einem Salz davon, worin $X_3$ Halogen oder Niederalkoxy bedeutet, umsetzt oder

e) zur Herstellung einer Verbindung der Formel I, eines Tautomeren und/oder Salzes davon, worin $R_2$ von Wasserstoff verschieden ist, in einer Verbindung der Formel

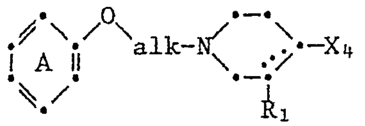

(VI)

oder einem Salz davon, worin $X_4$ einen in $R_2$ überführbaren Rest bedeutet, $X_4$ überführt oder

f) insbesondere zur Herstellung einer Verbindung der Formel I, eines Tautomeren und/oder Salzes davon, worin $R_2$ Wasserstoff bedeutet, eine Verbindung der Formel

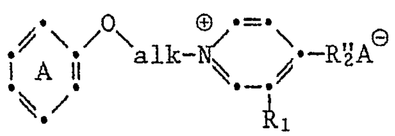

(VII),

worin $A^{\ominus}$ für das Anion einer Säure steht, und $R_2''$ Wasserstoff, veräthertes, verestertes oder geschütztes Hydroxy oder acyliertes oder geschütztes Amino bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet, jeweils eine verfahrensgemäss erhältliche freie Verbindung der Formel I in ein Salz überführt und/oder jeweils ein verfahrensgemäss erhältliches Salz in die freie Verbindung der Formel I oder in ein Anderes Salz umwandelt.

13. Eine Verbindung gemäss einem der Ansprüche 1 bis 11 oder ein Tautomeres und/oder ein pharmazeutisch verwendbares Salz davon zur Anwendung in einem Verfahren zur therapeutischen und/oder prophylaktischen Behandlung des menschlichen oder tierischen Körpers.

14. Eine Verbindung gemäss einem der Ansprüche 2, 4, 6, 8 und 9 oder ein Tautomeres und/oder ein pharmazeutisch verwendbares Salz davon zur Anwendung in einem Verfahren zur therapeutischen und/oder prophylaktischen Behandlung des menschlichen oder tierischen Körpers.

15. Ein pharmazeutisches Präparat, als Wirkstoff enthaltend eine Verbindung gemäss einem der Ansprüche 1 bis 11 und 13 oder ein Tautomeres und/oder ein pharmazeutisch verwendbares Salz davon gegebenenfalls neben üblichen pharmazeutischen Hilfsstoffen.

16. Ein pharmazeutisches Präparat, als Wirkstoff enthaltend eine Verbindung gemäss einem der Ansprüche 2, 4, 6, 8, 9 und 14 oder ein Tautomeres und/oder ein pharmazeutisch verwendbares Salz davon gegebenenfalls neben üblichen pharmazeutischen Hilfsstoffen.

17. Verwendung einer Verbindung gemäss einem der Ansprüche 1 bis 11 und 13 oder eines Tautomeren und/oder eines pharmazeutisch verwendbaren Salzes davon zur Herstellung eines pharmazeutischen Präparats, z.B. eines Nootropikums.

18. Das Verfahren der Beispiele 1 bis 36.

19. Die nach dem Verfahren gemäss einem der Ansprüche 12 und 18 verfahrensgemäss verwendeten neuen Ausgangsstoffe, gebildeten neuen Zwischenprodukte und erhältlichen neuen Endstoffe.

Patentansprüche für die folgenden Vertragsstaaten AT, ES und GR

1. Verfahren zur Herstellung einer Verbindung der Formel

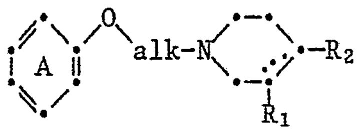

(I),

worin $R_1$ Niederalkoxycarbonyl, Carbamyl, N-Niederalkylcarbamyl, N,N-Diniederalkylcarbamyl oder gegebenenfalls acyliertes Hydroxymethyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder eines Tautomeren und/oder Salzes davon, dadurch gekennzeichnet, dass man

a) eine Verbindung der Formel

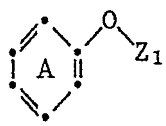

(IIa)

oder ein Salz davon mit einer Verbindung der Formel

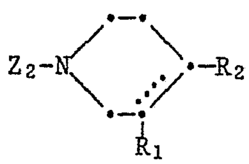

(IIb),

einem Tautomeren und/oder Salz davon, wobei entweder $Z_1$ eine Gruppe alk-$X_1$ und $Z_2$ Wasserstoff ist, oder $Z_1$ Wasserstoff und $Z_2$ eine Gruppe alk-$X_1$ darstellt, jeweils mit $X_1$ als Hydroxy oder reaktionsfähigem veresterten Hydroxy, umsetzt oder

b) in einer Verbindung der Formel

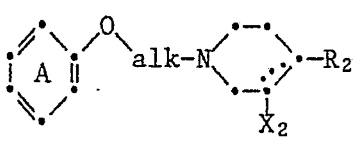

(III) ,

einem Tautomeren und/oder Salz davon, worin $X_2$ einen in $R_1$ überführbaren Rest bedeutet, $X_2$ in $R_1$ überführt oder

c) zur Herstellung einer Verbindung der Formel I, eines Tautomeren und/oder Salzes davon, worin $R_2$ Hydroxy oder Amino bedeutet, und worin $R_1$ insbesondere Niederalkoxycarbonyl bedeutet, eine Verbindung der Formel

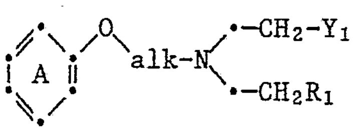

(IV),

worin $Y_1$ eine Gruppe der Formel -CH=$R_2'$ , -C(Y )=$R_2'$ , -CH($Y_2$ )-$R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', eines Tautomeren und/oder Salzes davon, worin $R_2'$ Oxo oder Imino bedeutet und die gestrichelte Linie zum Ausdruck bringen soll, dass eine Einfachbindung vorliegt, und worin $R_1$ insbesondere Niederalkoxycarbonyl bedeutet, eine Verbindung der Formel

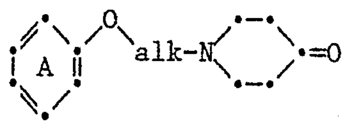

(Va)

oder ein Tautomeres oder jeweils ein Salz davon mit einer Verbindung der Formel
$X_3$ -$R_1$ (Vb)
oder einem Salz davon, worin $X_3$ Halogen oder Niederalkoxy bedeutet, umsetzt oder

e) zur Herstellung einer Verbindung der Formel I, eines Tautomeren und/oder Salzes davon, worin $R_2$ von Wasserstoff verschieden ist, in einer Verbindung der Formel

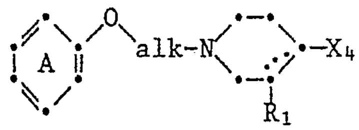

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I, eines Tautomeren und/oder Salzes davon, worin $R_2$ Wasserstoff bedeutet, eine Verbindung der Formel

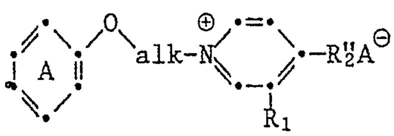

(VII),

worin $A^\ominus$ für das Anion einer Säure steht, und $R_2''$ Wasserstoff, veräthertes, verestertes oder geschütztes Hydroxy oder acyliertes oder geschütztes Amino bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung in eine andere Verbindung der Formel I überführt, jeweils ein verfahrensgemäss erhältliches Isomerengemisch in die Komponenten auftrennt, jeweils ein verfahrensgemää erhältliches Enantiomeren- bzw. Diastereomerengemisch in die Enantiomeren bzw. Diastereomeren aufspaltet, jeweils eine verfahrensgemäss erhältliche freie Verbindung der Formel I in ein Salz überführt und/oder jeweils ein verfahrensgemäss erhältliches Salz in die freie Verbindung der Formel I oder in ein anderes Salz umwandelt.

2. Verfahren gemäss Anspruch 1 zur Herstellung einer Verbindung der Formel I, worin $R_1$ Niederalkoxycarbonyl, Carbamyl, N-Niederalkylcarbamyl oder N,N-Diniederalkylcarbamyl bedeutet, $R_2$ und alk die angegebenen Bedeutungen haben, der Ring A unsubstituiert oder ein- oder mehrfach wie angegeben substituiert ist und die gestrichelte Linie zum Ausdruck bringen soll, dass eine Einfach- oder eine Doppelbindung vorliegt, eines Tautomeren und/oder Salzes davon, dadurch gekennzeichnet, dass man in einer Verbindung der Formel I, worin $R_1$ Hydroxymethyl oder acyliertes Hydroxymethyl darstellt, zunächst gegebenenfalls vorliegendes acyliertes Hydroxymethyl $R_1$ in Hydroxymethyl $R_1$ überführt, sodann Hydroxymethyl $R_1$ zu Carboxy oxidiert und die erhaltene Carboxygruppe anschliessend zu Niederalkoxycarbonyl $R_1$ verestert oder zu Carbamyl, N-Niederalkylcarbamyl oder N,N-Diniederalkylcarbamyl $R_1$ amidiert und jeweils eine gegebenenfalls vorhandene Schutzgruppe abspaltet und jeweils gewünschtenfalls eine verfahrensgemäss oder auf andere Weise erhältliche Verbindung in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet, jeweils eine verfahrensgemäss erhältliche freie Verbindung der Formel I in ein Salz überführt und/oder jeweils ein verfahrensgemäss erhältliches Salz in die freie Verbindung der Formel I oder in ein anderes Salz umwandelt.

3. Verfahren gemäss Anspruch 1 oder 2 zur Herstellung einer Verbindung der Formel I, worin $R_1$ Niederalkoxycarbonyl, Carbamyl, N-Niederalkylcarbamyl oder N,N-Diniederalkylcarbamyl bedeutet, $R_2$ Wasserstoff, eine gegebenenfalls verätherte oder acylierte Hydroxygruppe oder eine gegebenenfalls acylierte Aminogruppe darstellt, alk Niederalkylen bedeutet, der Ring A unsubstituiert oder ein-oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach oder eine Doppelbindung vorliegt, oder eines Tautomeren und/oder Salzes davon.

4. Verfahren gemäss Anspruch 1 oder 2 zur Herstellung einer Verbindung der Formel I, worin $R_1$ Niederalkoxycarbonyl, Carbamyl, N-Niederalkylcarbamyl, N,N-Diniederalkylcarbamyl, Hydroxymethyl,

Niederalkanoyloxymethyl, Niederalkansulfonyloxymethyl, Benzoyloxymethyl oder Pyridoyloxymethyl bedeutet, $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Niederalkansulfonylamino, Benzoylamino oder Pyridoylamino darstellt, alk Niederalkylen, das die in Formel I eingezeichneten Heteroatome O und N in erster Linie durch 2 bis und mit 4 C-Atome überbrückt, bedeutet, der Ring A unsubstituiert ist oder ein-, zwei-oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder eines Tautomeren und/oder Salzes davon.

5. Verfahren gemäss Anspruch 1 oder 2 zur Herstellung einer Verbindung der Formel I, worin $R_1$ Niederalkoxycarbonyl, Carbamyl, N-Niederalkylcarbamyl oder N,N-Diniederalkylcarbamyl bedeutet, $R_2$ Wasserstoff, Hydroxy, Niederalkoxy, Benzyloxy, Niederalkanoyloxy, Niederalkansulfonyloxy, Benzoyloxy, Pyridoyloxy, Amino, Niederalkanoylamino, Benzoylamino oder Pyridoylamino darstellt, alk Niederalkylen, das die beiden in Formel I eingezeichneten Heteroatome O und N in erster Linie durch 2 bis und mit 3 C-Atome überbrückt, bedeutet, der Ring A unsubstituiert ist oder ein-, zwei-oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder eines Tautomeren und/oder Salzes davon.

6. Verfahren gemäss Anspruch 1 oder 2 zur Herstellung einer Verbindung der Formel I, worin $R_1$ $C_1$ -$C_4$ -Alkoxycarbonyl, Carbamyl, N-$C_1$ -$C_4$ -Alkylcarbamyl, N,N-Di-$C_1$ -$C_4$ -Alkylcarbamyl, Hydroxymethyl oder $C_2$ -$C_5$ -Alkanoyloxymethyl bedeutet, $R_2$ Wasserstoff, Hydroxy oder Amino darstellt, alk für $C_1$ -$C_4$ -Alkylen, das die beiden in Formel I eingezeichneten Heteroatome O und N in erster Linie durch 2 bis und und mit 4 C-Atome überbrückt, steht, der Ring A unsubstituiert oder ein-, zwei- oder mehrfach durch $C_1$ -$C_4$ -Alkoxy, Cyano, Halogen mit einer Atomnummer bis und mit 35, $C_1$ -$C_4$ -Alkyl, und/oder Trifluormethyl substituiert ist und die gestrichelte Linie zum Ausdruck bringen soll, dass eine Einfach- oder eine Doppelbindung vorliegt, oder eines Tautomeren und/oder Salzes davon.

7. Verfahren gemäss Anspruch 1 oder 2 zur Herstellung einer Verbindung der Formel I, worin $R_1$ $C_1$ -$C_4$ -Alkoxycarbonyl oder Carbamyl bedeutet, $R_2$ Wasserstoff oder Hydroxy bedeutet, alk für $C_1$ -$C_4$ -Alkylen, das die beiden in Formel I eingezeichneten Heteroatome O und N in erster Linie durch 2 bis und mit 3 C-Atome überbrückt, steht, der Ring A unsubstituiert oder ein-, zwei- oder mehrfach durch $C_1$ -$C_4$ -Alkoxy, Cyano, Halogen mit einer Atomnummer bis und mit 35, $C_1$ -$C_4$ -Alkyl und/oder Trifluormethyl substituiert ist und die gestrichelte Linie zum Ausdruck bringen soll, dass eine Doppelbindung vorliegt, oder eines Tautomeren und/oder Salzes davon.

8. Verfahren gemäss Anspruch 1 oder 2 zur Herstellung einer Verbindung der Formel I, worin $R_1$ $C_1$ -$C_4$ -Alkoxycarbonyl oder Carbamyl bedeutet, $R_2$ Wasserstoff oder Hydroxy darstellt, alk für $C_1$ -$C_4$ -Alkylen, das die beiden in Formel I eingezeichneten Heteroatome O und N in erster Linie durch 2 bis und mit 3 C-Atome überbrückt, steht, der Ring A einfach durch $C_1$ -$C_4$ -Alkoxy, $C_1$ -$C_4$ -Alkyl oder Trifluormethyl oder zweifach durch Halogen mit einer Atomnummer bis und mit 35 oder $C_1$ -$C_4$ -Alkyl oder $C_1$ -$C_4$ -Alkoxy sowie $C_1$ -$C_4$ -Alkyl substituiert ist und die gestrichelte Linie zum Ausdruck bringen soll, dass eine Einfach- oder eine Doppelbindung vorliegt, oder eines Tautomeren und/oder Salzes davon.

9. Verfahren gemäss Anspruch 1 oder 2 zur Herstellung von 1-[2-(2,4-Dichlorphenoxy)äthyl]-1,2,5,6-tetrahydro-pyridin-3-carbonsäuremethylester, 1-[2-(o-Methoxyphenoxy)äthyl]-1,2,5,6-tetrahydro-pyridin-3-carbonsäuremethylester, 1-[2-(m-Trifluormethylphenoxy)- äthyl]-1,2,5,6-tetrahydro-pyridin-3-carbonsäuremethylester und 1-[2-(p-Methylphenoxy)äthyl]-1,2,5,6-tetrahydro-pyridin-3-carbonsäuremethylester oder jeweils eines Salzes davon.

10. Verfahren gemäss Anspruch 1 oder 2 zur Herstellung von 1-[2-(m-Methoxyphenoxy)äthyl]-1,2,5,6-tetrahydro-pyridin-3-carbonsäuremethylester, 1-[2-(p-Chlorphenoxy)äthyl]-1,2,5,6-tetrahydro-pyridin-3-carbonsäuremethylester, 1-[2-(p-Methoxyphenoxy)äthyl]-1,2,5,6-tetrahydro-pyridin-3-carbonsäuremethylester, 1-[2-(p-Cyanophenoxy)-äthyl]-1,2,5,6-tetrahydro-pyridin-3-carbonsäuremethylester, 1-[2-(p-Chlorphenoxy)äthyl]-4-hydroxy-1,2,5,6-tetrahydro-pyridin-3-carbonsäuremethylester bzw. 1-[2-(p-Chlorphenoxy)äthyl]-4-oxo-piperidin-3-carbonsäuremethylester, 1-[2-(p-Methoxyphenoxy)äthyl]-4-hydroxy-1,2,5,6-tetrahydro-pyridin-3-carbonsäuremethylester bzw. 1-[2-(p-Methoxyphenoxy)-äthyl]-4-oxo-piperidin-3-carbonsäuremethylester, 1-[2-(o-Methoxyphenoxy)äthyl]-4-hydroxy-1,2,5,6-tetrahydro-pyridin-3-carbonsäuremethylester bzw. 1-[2-(o-Methoxyphenoxy)-äthyl]-4-oxo-piperidin-3-carbonsäuremethylester und 4-Hydroxy-1-[3-(m-trifluormethylphenoxy)propyl]-1,2,5,6-tetrahydropyridin-3-carbonsäuremethylester bzw. 4-Oxo-1-[3-(m-trifluormethylphenoxy)propyl]-piperidin-3-carbonsäuremethylester oder jeweils eines Salzes davon.

11. Verfahren gemäss Anspruch 1 oder 2 zur Herstellung von 4-Hydroxy-1-[2-(p-methylphenoxy)äthyl]-1,2,5,6-tetrahydro-pyridin-3-carbonsäuremethylester bzw. 1-[2-(p-Methylphenoxy)äthyl]-4-oxo-piperidin-3-carbonsäuremethylester, 4-Hydroxy-1-[2-(m-trifluormethylphenoxy)äthyl]-1,2,5,6-tetrahydro-pyridin-3-carbonsäuremethylester bzw. 4-Oxo-1-[2-(m-trifluormethylphenoxy)äthyl]-piperidin-3-carbonsäuremethylester, 1-[2-(m-Trifluormethylphenoxy)äthyl]-piperidin-3-carbonsäureäthylester, 1-[3-(m-Trifluormethylphenoxy)propyl]-piperidin-3-carbonsäureäthylester, 1-[2-(p-Methylphenoxy)äthyl]-piperidin-3-carbonsäureäthylester, 1-[2-(2-Methoxy-5-methyl-phenoxy)äthyl]-piperidin-3-carbonsäureäthylester, 1-[2-(3,4-Dichlorphenoxy)äthyl]-piperidin-3-carbonsäure-

äthylester, 1-[2-(3,4- Dimethylphenoxy)äthyl]-piperidin-3-carbonsäureäthylester und 1-[2-(p-Methylphenoxy)äthyl]-piperidin-3-carbonsäureamid oder jeweils eines Salzes davon.

12. Verfahren gemäss Anspruch 1 oder 2 zur Herstellung von 1-[2-(p-Methylphenoxy)äthyl]-piperidin-3-carbonsäuremethylester, 1-[3-(m-Trifluormethylphenoxy)propyl]-1,2,5,6-tetrahydro-pyridin-3-carbonsäuremethylester, 1-[2-(2,4-Dichlorphenoxy)äthyl]-piperidin-3-carbonsäureäthylester, 1-[2-(3,4-Dichlorphenoxy)äthyl]-1,2,5,6-tetrahydro-pyridin-3-carbonsäuremethylester, 1-[2-(2,5-Dimethylphenoxy)äthyl]-piperidin-3-carbonsäureäthylester, 1-[2-(2,5-Dimethylphenoxy)äthyl]-1,2,5,6-tetrahydro-pyridin-3-carbonsäuremethylester, 1-[2-(m-Methylphenoxy)äthyl]-piperidin-3-carbonsäureäthylester, 1-[2-(m-Methylphenoxy)äthyl]-1,2,5,6-tetrahydro-pyridin-3-carbonsäuremethylester, 1-[2-(o-Methylphenoxy)äthyl]-piperidin-3-carbonsäureäthylester, 1-[2-(o-Methylphenoxy)äthyl]-1,2,5,6-tetrahydro-pyridin-3-carbonsäuremethylester, 1-[2-(3,4-Dimethylphenoxy)äthyl]-1,2,5,6-tetrahydro-pyridin-3-carbonsäuremethylester, 1-[2-(p-Methylphenoxy)äthyl]-piperidin-3-carbonsäure-n-butylester, 1-[2-(m-Trifluormethylphenoxy)äthyl]-piperidin-3-carbonsäureamid, 1-[2-(m-Trifluormethylphenoxy)äthyl]-piperidin-3-carbonsäure-n-butylester, 1-[2-(2,4-Dichlorphenoxy)äthyl]-piperidin-3-carbonsäureamid, 1-[3-(m-Trifluormethylphenoxy)propyl]-piperidin-3-carbonsäureamid, 1-[2-(3,4-Dimethylphenoxy)äthyl]-piperidin-3-carbonsäureamid, 1-[4-(m-Trifluormethylphenoxy)butyl]-piperidin-3-carbonsäureäthylester, 1-[2-(3,4-Dimethylphenoxy)äthyl]-piperidin-3-carbonsäure-N,N-diäthylamid, 1-[2-(m-Trifluormethylphenoxy)äthyl]-piperidin-3-carbonsäure-N,N-diäthylamid, 1-[2-(p-Methylphenoxy)äthyl]-piperidin-3-carbonsäure-N,N-diäthylamid, 1-[2-(2,4-Dichlorphenoxy)äthyl]-piperidin-3-carbonsäure-N,N-diäthylamid, 4-Amino-1-[2-(p-methylphenoxy)äthyl]-piperidin-3-carbonsäureamid, 1-[2-(p-Trifluormethylphenoxy)äthyl]-1,2,5,6-tetrahydro-pyridin-3-carbonsäuremethylester, 1-[2-(5-Methoxy-2-methyl-phenoxy)äthyl]-1,2,5,6-tetrahydro-pyridin-3-carbonsäuremethylester, 4-Hydroxy-1-[2-(5-methoxy-2-methylphenoxy)äthyl]-1,2,5,6-tetrahydro-pyridin-3-carbonsäuremethylester bzw. 1-[2-(5-Methoxy-2-methyl-phenoxy)äthyl]-4-oxo-piperidin-3- carbonsäuremethylester, 4-Hydroxy-1-[2-(2,5-dimethoxyphenoxy)äthyl]-1,2,5,6-tetrahydro-pyridin-3-carbonsäuremethylester bzw. 1-[2-(2,5-Dimethoxyphenoxy)-äthyl]-4-oxo-piperidin-3-carbonsäuremethylester, 1-[2-(2,5-Dimethoxyphenoxy)äthyl]-1,2,5,6-tetrahydro-pyridin-3-carbonsäuremethylester, 1-[4-(m-Trifluormethylphenoxy)butyl]-piperidin-3-carbonsäureamid und 1-[2-(p-Cyanophenoxy)äthyl]-piperidin-3-carbonsäureamid oder jeweils eines Salzes davon.

13. Verfahren gemäss Anspruch 1 zur Herstellung von 1-[2-(p-Methylphenoxy)äthyl]-3-hydroxymethylpiperidin und 1-[4-(m-Trifluormethylphenoxy)butyl]-3-hydroxymethyl-piperidin oder jeweils eines Salzes davon.

14. Verfahren zur Herstellung eines pharmazeutischen Präparates, dadurch gekennzeichnet, dass man

a) eine Verbindung der Formel

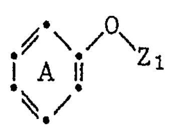

(IIa)

oder ein Salz davon mit einer Verbindung der Formel

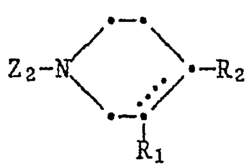

(IIb),

einem Tautomeren und/oder Salz davon, wobei entweder $Z_1$ eine Gruppe alk-$X_1$ und $Z_2$ Wasserstoff ist, oder $Z_1$ Wasserstoff und $Z_2$ eine Gruppe alk-$X_1$ darstellt, jeweils mit $X_1$ als Hydroxy oder reaktionsfähigem veresterten Hydroxy, umsetzt oder

b) in einer Verbindung der Formel

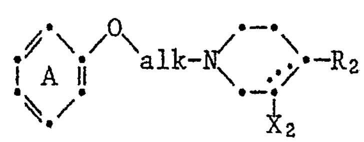

(III) ,

einem Tautomeren und/oder Salz davon, worin $X_2$ einen in $R_1$ überführbaren Rest bedeutet, $X_2$ in $R_1$ überführt oder

c) zur Herstellung einer Verbindung der Formel I, eines Tautomeren und/oder Salzes davon, worin $R_2$ Hydroxy oder Amino bedeutet, und worin $R_1$ insbesondere Niederalkoxycarbonyl bedeutet,

eine Verbindung der Formel

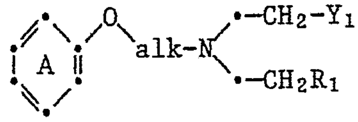

(IV),

worin $Y_1$ eine Gruppe der Formel $-CH=R_2'$, $-C(Y_2)=R_2'$, $-CH(Y_2)-R_2$ oder Cyano darstellt, wobei $R_2'$ Oxo oder Imino bedeutet und $Y_2$ einen abspaltbaren Rest darstellt, oder ein Salz davon cyclisiert oder

d) zur Herstellung einer Verbindung der Formel I', eines Tautomeren und/oder Salzes davon, worin $R_2'$ Oxo oder Imino bedeutet und die gestrichelte Linie zum Ausdruck bringen soll, dass eine Einfachbindung vorliegt, und worin $R_1$ insbesondere Niederalkoxycarbonyl bedeutet, eine Verbindung der Formel

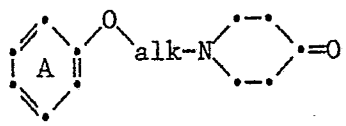

(Va)

oder ein Tautomeres oder jeweils ein Salz davon mit einer Verbindung der Formel

$X_3-R_1$ (Vb)

oder einem Salz davon, worin $X_3$ Halogen oder Niederalkoxy bedeutet, umsetzt oder

e) zur Herstellung einer Verbindung der Formel I, eines Tautomeren und/oder Salzes davon, worin $R_2$ von Wasserstoff verschieden ist, in einer Verbindung der Formel

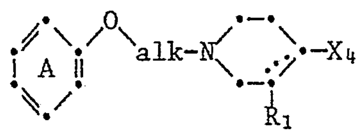

(VI)

oder einem Salz davon, worin $X_4$ einen in $R_2$ überführbaren Rest bedeutet, $X_4$ in $R_2$ überführt oder

f) insbesondere zur Herstellung einer Verbindung der Formel I, eines Tautomeren und/oder Salzes davon, worin $R_2$ Wasserstoff bedeutet, eine Verbindung der Formel

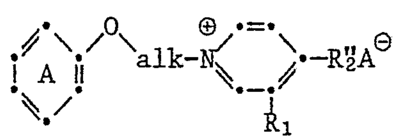

(VII),

worin $A^\ominus$ für das Anion einer Säure steht, und $R_2''$ Wasserstoff, veräthertes, verestertes oder geschütztes Hydroxy oder acyliertes oder geschütztes Amino bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung in eine andere Verbindung der Formel I überführt, jeweils ein verfahrensgemäss erhältliches Isomerengemisch in die Komponenten auftrennt, jeweils ein verfahrensgemäss erhältliches Enantiomeren- bzw. Diastereomerengemisch in die Enantiomeren bzw. Diastereomeren aufspaltet, jeweils eine verfahrensgemäss erhältlich freie Verbindung der Formel I in ein Salz überführt und/oder jeweils ein verfahrensgemäss erhältliches Salz in die freie Verbindung der Formel I oder in ein anderes Salz unwandelt und eine jeweils erhaltene Verbindung der Formel

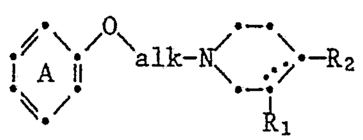

(I),

worin $R_1$ Niederalkoxycarbonyl, Carbamyl, N-Niederalkylcarbamyl, N,N-Diniederalkylcarbamyl oder

gegebenenfalls acyliertes Hydroxymethyl bedeutet, $R_2$ Wasserstoff, eine gegebenenfalls verätherte oder acylierte Hydroxygruppe oder eine gegebenenfalls acylierte Amino gruppe darstellt, alk Niederalkylen bedeutet, der Ring A unsubstituiert oder ein- oder mehrfach durch Hydroxy, Niederalkoxy, Niederalkanoyloxy, Cyano, Halogen, Niederalkyl und/oder Trifluormethyl substituiert ist und die gestrichelte Linie zum Ausdruck bringen soll, dass eine Einfach- oder eine Doppelbindung vorliegt, oder ein jeweils erhaltenes Tautomeres und/oder ein jeweils erhaltenes pharmazeutisch verwendbares Salz davon mit üblichen pharmazeutischen Hilfsstoffen vermischt.

15. Verfahren zur Herstellung eines pharmazeutischen Präparates, dadurch gekennzeichnet, dass man eine Verbindung, erhältlich gemäss einem der Ansprüche 1 bis 13, oder ein Tautomeres und/oder ein pharmazeutisch verwendbares Salz davon mit üblichen pharmazeutischen Hilfsstoffen vermischt.

16. Verfahren zur Herstellung eines pharmazeutischen Präparates, dadurch gekennzeichnet, dass man eine Verbindung, erhältlich gemäss einem der Ansprüche 3, 5, 7, 9 und 10, oder ein Tautomeres und/oder ein pharmazeutisch verwendbares Salz davon mit üblichen pharmazeutischen Hilfsstoffen vermischt.

17. Verwendung einer Verbindung, erhältlich gemäss einem der Ansprüch 1 bis 13, oder eines Tautomeren und/oder eines pharmazeutisch verwendbaren Salzes davon zur Herstellung eines pharmazeutischen Präparates, z.B. eines Nootropikums.

18. Das Verfahren der beispiele 1 bis 36.

19. Die nach dem Verfahren gemäss einem der Ansprüche 1 bis 13 und 18 verfahrensgemäss verwendeten neuen Ausgangsstoffe, gebildeten neuen Zwischenprodukte und erhältlichen neuen Endstoffe.

Europäisches Patentamt

# EUROPÄISCHER RECHERCHENBERICHT

Nummer der Anmeldung

EP 87 81 0349

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 929 813 (HIGUCHI et al.)<br>* Zusammenfassung; Ansprüche *<br>--- | 1-19 | C 07 D 211/60<br>C 07 D 211/78<br>A 61 K 31/445 |
| Y | EP-A-0 066 456 (SMITH KLINE BECKMAN CORP.)<br>* Insgesamt *<br>----- | 1-19 | |
| | | | **RECHERCHIERTE SACHGEBIETE (Int. Cl.4)** |
| | | | C 07 D 211/00 |

Der vorliegende Recherchenbericht wurde für alle Patentansprüche erstellt.

| Recherchenort | Abschlußdatum der Recherche | Prüfer |
|---|---|---|
| DEN HAAG | 22-09-1987 | MAISONNEUVE J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82